# EUROPEAN PATENT APPLICATION

(11) **EP 4 800 038 A1**
(43) Date of publication of application: **02.09.2026**
(21) Application number: 24881742.1
(22) Date of filing: 25.10.2024
(51) Int. Cl.: C07K 19/00, C12N 5/10, C12N 15/62, A61K 39/00, A61K 45/06, A61P 35/00, A61P 35/02

(54) **THERAPEUTIC USE OF CHIMERIC ANTIGEN RECEPTOR TARGETING CD19**

(30) Priority: 26.10.2023 CN 202311404971; 09.10.2024 CN 202411406524
(71) Applicant: Shanghai Simnova Biotechnology Co., Ltd., Shanghai 201321 (CN)
(72) Inventor: XU, Zupeng, Shanghai 201318 (CN); GAO, Fangjie, Shanghai 201318 (CN); WANG, Chao, Shanghai 201318 (CN); ZHOU, Ling, Shanghai 201318 (CN); CHEN, Qiwei, Shanghai 201318 (CN); WEN, Cheng, Shanghai 201318 (CN); CAO, Zhuoxiao, Shanghai 201318 (CN)
(74) Representative: Richly & Ritschel Patentanwälte PartG mbB
(86) International application number: PCT/CN2024/127354
(87) International publication number: WO 2025/087382

(57) **Abstract**

Provided are a chimeric antigen receptor targeting CD19 and therapeutic and pharmaceutical uses thereof in B cell-driven related diseases such as tumors or autoimmune diseases, and a method for treating diseases such as tumors or autoimmune diseases.

## Description

### CROSS-REFERENCE TO RELATED APPLICATIONS

The present application claims priority to and related benefit of Chinese Patent Application No. 202311404971.8 filed on October 26, 2023 and Chinese Patent Application No. 202411406524.0 filed on October 9, 2024, the contents of which are incorporated herein by reference in their entirety.

### TECHNICAL FIELD

The present application generally relates to the field of bioengineering and cell therapy, and in particular, to a chimeric antigen receptor targeting CD19 and therapeutic use thereof in a tumor or an autoimmune disease.

### BACKGROUND

B-cell malignancy is a malignancy characterized by abnormal proliferation of B cells, and relatively common B-cell malignancies include acute precursor B-lymphocytic leukemia (acute lymphoblastic leukemia, ALL), non-Hodgkin lymphoma (NHL), B-cell chronic lymphocytic leukemia (chronic lymphocytic leukemia, CLL), hairy cell leukemia, acute common B-lymphocytic leukemia, and the like. In the process of searching for novel therapies for B-cell malignancies, CAR T therapies targeting the cell surface antigen CD19 of B-cell malignancies are a major research advance at present and have achieved significant clinical effects.

Moreover, the dysfunction of B cells may also be highly associated with the pathogenesis of autoimmune diseases. For example, systemic lupus erythematosus (SLE) is an autoimmune disease involving multiple organs, the main pathogenesis of which is autoimmune reaction caused by immune imbalance, and the most critical factor is the dysregulation of the activation of autoreactive B cells. CD19 serves as a characteristic antigen on the surface of B cells. Targeting CD19 and further exploring the function of B cells may provide a new therapeutic strategy for autoimmune diseases. There is a need to develop effective and safe cell products based on chimeric antigen receptors that specifically target CD19.

### SUMMARY

The present application provides an immune cell comprising a chimeric antigen receptor targeting CD19, a corresponding pharmaceutical composition, and use thereof in the treatment of a related disease driven by B cells, such as a tumor or an autoimmune disease.

In a first aspect, the present application provides an immune effector cell comprising a chimeric antigen receptor targeting CD19, wherein the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3, wherein:
(1) the HCDR1-3 have the sequences set forth in SEQ ID NOs: 5-7, respectively, and the LCDR1-3 have the sequences set forth in SEQ ID NOs: 8-10, respectively; or
(2) the HCDR1-3 have at most 0-3 mutations compared with each corresponding CDR in the above (1), respectively, and/or the LCDR1-3 have at most 0-3 mutations compared with each corresponding CDR in the above (1), respectively.

In a second aspect, the present application provides a pharmaceutical composition comprising the immune effector cell according to the first aspect and a pharmaceutically acceptable carrier.

In a third aspect, the present application provides the immune effector cell according to the first aspect or the pharmaceutical composition according to the second aspect for use in treating a disease, wherein the disease is a B cell-driven disease, preferably a disease driven by a CD19-positive B cell. In a fourth aspect, the present application provides use of the immune effector cell according to the first aspect or the pharmaceutical composition according to the second aspect in the preparation of a medicament for treating a disease, wherein the disease is a B cell-driven disease, preferably a disease driven by a CD19-positive B cell.

In a fifth aspect, the present application provides a method for treating a disease, which comprises administering to a subject in need an effective amount of the immune effector cell according to the first aspect or the pharmaceutical composition according to the second aspect, wherein the disease is a B cell-driven disease, preferably a disease driven by a CD19-positive B cell.

In some embodiments, the disease according to the third to fifth aspects described above is a tumor or cancer.In a preferred embodiment, the tumor or cancer is a CD19-related tumor or cancer.In a more preferred embodiment, the tumor or cancer is a B-cell malignancy, such as acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

In some embodiments, the disease according to the third to fifth aspects described above is an autoimmune disease, including systemic lupus erythematosus (SLE), inflammatory bowel disease (IBD), multiple sclerosis (MS), rheumatoid arthritis (RA), type I diabetes (T1DM), Sjogren's syndrome, myositis, vasculitis, scleroderma, and the like. In some preferred embodiments, the disease is moderate to severe active systemic lupus erythematosus, refractory idiopathic myositis, refractory granulomatosis with polyangiitis, or systemic sclerosis.

The inventors of the present application developed a novel chimeric antigen receptor targeting CD19 and use thereof through a great deal of research and tests, and provided a chimeric antigen receptor targeting CD19 and a corresponding nucleic acid molecule, a vector, an immune effector cell, a preparation method and a product thereof, a pharmaceutical composition, therapeutic use, pharmaceutical use, and a method for treating a tumor or cancer or an autoimmune disease. Each invention of the present application realizes at least one of the following beneficial effects: (1) having a specific killing effect on CD19 positive tumor cells and/or having an inhibition effect on the growth of CD19-related tumors; (2) reducing the immunogenicity through humanizing the chimeric antigen receptor targeting CD19; (3) having application prospects for "shelf-type" therapeutic products in the case of an exemplary effector cell NK cell; (4) being expected to have a relatively good clinical therapeutic effect on CD19-positive tumors, such as acute B-lymphocytic leukemia (B-ALL), non-Hodgkin's lymphoma (NHL), chronic lymphocytic leukemia (CLL)/small lymphocytic lymphoma (SLL), or autoimmune diseases, such as systemic lupus erythematosus, myositis, scleroderma, vasculitis, and the like.

### BRIEF DESCRIPTION OF THE DRAWINGS

FIG. 1 shows a schematic diagram of the structure of a chimeric antigen receptor targeting CD19.
FIG. 2A shows the expansion of different CD19 CAR-NKs, wherein UT represents untreated.
FIG. 2B shows the CAR cell positive rate of different CD19 CAR-NKs during expansion culture.
FIGs. 3A and 3B show the killing effect of different CD19 CAR-NKs on Raji cells, wherein FIG. 3A shows the results at 0 h of thawing, and FIG. 3B shows the results at 24 h after thawing.
FIGs. 4A and 4B show the tumor imaging results of the Nalm6 xenograft model in animals after receiving different CD19 CAR-NK treatments, wherein FIG. 4A shows the results on days 5-26 and FIG. 4B shows the results on days 30-33.
FIG. 4C shows the photon count results of the tumor imaging of the Nalm6 xenograft model in animals after receiving different CD19 CAR-NK treatments (on day 19, animals in the negative control group started to die, so the photon count data were only recorded up to day 19).
FIG. 4D shows the body weight results of the animals of the Nalm6 xenograft model after receiving different CD19 CAR-NK treatments (on day 19, animals in the negative control group started to die, so the body weight data were only recorded up to day 19).
FIG. 4E shows the survival curves of the animals of the Nalm6 xenograft model after receiving different CD19 CAR-NK treatments.
FIG. 5A shows the proportion of CD56⁺CAR19⁺CD3⁻ cells in peripheral blood samples obtained from the animals of the Nalm6 xenograft model receiving different CD19 CAR-NK treatments.
FIG. 5B shows the proportion of CD56⁺CAR19⁻CD3⁻ cells in peripheral blood samples obtained from the animals of the Nalm6 xenograft model receiving different CD19 CAR-NK treatments.
FIG. 6 shows a summary of efficacy assay of the Nalm6 xenograft model for different CD19 CAR-NK treatment groups.
FIG. 7A shows the cell viability statistics after in vitro short-acting killing of B cells from peripheral blood of SLE patients by CD19 CAR-NK.
FIG. 7B shows the cell viability statistics after in vitro multiple-run killing of B cells from peripheral blood of SLE patients by CD19 CAR-NK.
FIG. 8 shows a flow chart of a preliminary clinical study protocol for the treatment of a B-cell malignancy with CD19 CAR-NK.
FIG. 9 shows a flow chart of a preliminary clinical study protocol for the treatment of SLE with CD19 CAR-NK.

### DETAILED DESCRIPTION

### Terms and Definitions

Unless otherwise defined herein, scientific and technical terms used in correlation with the present application shall have the meanings that are commonly understood by those skilled in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

The terms "include", "comprise", and "have" herein are used interchangeably and are intended to indicate the inclusion of a solution, implying that there may be elements other than those listed in the solution. Meanwhile, it should be understood that the descriptions "include", "comprise", and "have" used herein also provide the solution of "consist of". Illustratively, "a composition, comprising A and B" should be interpreted as the following technical solution: a composition consisting of A and B, and a composition containing other components in addition to A and B, all of which fall within the scope of the "composition" described above.

The term "and/or" used herein includes the meanings of "and", "or", and "all or any other combination of elements linked by the term".

Although the numerical ranges and parameter approximations are shown in the broad scope of the present application, the numerical values shown in the specific examples are recorded as accurately as possible. However, any numerical values inherently contain certain errors necessarily resulting from the standard deviations found in their respective measurements. In addition, all ranges disclosed herein should be understood to encompass any and all subranges subsumed therein. For example, a recorded range of "1 to 10" should be considered to include any and all subranges between the minimum value of 1 and the maximum value of 10 (including the endpoints); that is, all subranges starting with a minimum value of 1 or greater, such as 1 to 6.1, and all subranges ending with a maximum value of 10 or less, such as 5.5 to 10. Additionally, any reference referred to as "incorporated herein" should be understood as being incorporated in its entirety.

As used herein, the term "CD19" is a 95 kDa type I transmembrane glycoprotein with a single transmembrane domain, a cytoplasmic C-terminus, and an extracellular N-terminus, wherein the extracellular domain consists of two C2-type Ig-like domains, and the highly conserved cytoplasmic domain consists of 242 amino acids and 9 tyrosine residues near the C-terminus. CD19 plays an important role in the establishment of the intrinsic B-cell signaling threshold by regulating B-cell receptor-dependent and -independent signaling. The CD19 antigen belongs to the immunoglobulin superfamily, which directly or indirectly recruits and binds to a variety of downstream protein kinases by interacting with other B cell receptors or surface molecules, including the Src family (Lyn and Fyn), Ras family, Abl, Btk, adapter molecules (Vav and Grb2), and PI3K protein kinases. CD19, together with the complement receptor CD21, the tetraspanin CD81 (TAPA-1), and CD225, is a major signaling component of multimolecular complexes on the surface of mature B cells.

As used herein, "chimeric antigen receptor (CAR)" refers to an artificial immune effector cell surface receptor engineered to be expressed on an immune effector cell and specifically bound to an antigen, which at least comprises (1) an extracellular antigen-binding domain, e.g., a variable heavy or light chain of an antibody, (2) a transmembrane domain that anchors the CAR into the immune effector cell, and (3) an intracellular signaling domain. The CAR is capable of redirecting T cells and other immune effector cells to a selected target, e.g., a cancer cell, in a non-MHC-restricted manner using the extracellular antigen-binding domain. The extracellular domain of a chimeric antigen receptor may also include a signal peptide and/or a hinge region. The intracellular domain of a chimeric antigen receptor may also include a costimulatory domain.

As used herein, the term "signal peptide" in the context of a chimeric antigen receptor refers to a fragment of a protein or polypeptide that is used to direct the protein or polypeptide into the secretory pathway and transfer them to the cell membrane and/or cell surface. A non-limiting example of a signal peptide is the CD8α signal peptide.

As used herein, the term "hinge region" in the context of a chimeric antigen receptor generally refers to any oligopeptide or polypeptide that functions to connect a transmembrane region and an antigen-binding region. In particular, the hinge region serves to provide greater flexibility and accessibility to the antigen-binding region. The hinge region may be derived in whole or in part from a natural molecule, e.g., an extracellular region derived in whole or in part from CD8, CD4, or CD28, or derived in whole or in part from an antibody constant region. Alternatively, the hinge region may be either a synthetic sequence corresponding to a naturally occurring hinge sequence, or a completely synthetic hinge sequence.

As used herein, the term "transmembrane (TM) region" in the context of a chimeric antigen receptor refers to a polypeptide structure that enables expression of the chimeric antigen receptor on the surface of an immune cell (e.g., a lymphocyte, an NK cell, or an NKT cell) and directs the cellular response of the immune cell against a target cell. The transmembrane domain may be natural or synthetic, and may be derived from any membrane-bound protein or transmembrane protein. The transmembrane domain can transduce a signal when the chimeric antigen receptor binds to a target antigen. A non-limiting example of a hinge region is the CD8 transmembrane region.

As used herein, the term "intracellular signaling domain" in the context of a chimeric antigen receptor refers to a protein portion that transduces an effector function signal and directs a cell to perform a specified function. The intracellular signaling domain is responsible for the intracellular primary signaling after the antigen-binding domain binds to an antigen, resulting in the activation of the immune cell and immune response. In other words, the intracellular signaling domain is responsible for activating at least one of the normal effector functions of the immune cell in which the CAR is expressed. Exemplary intracellular signaling domains include CD3ζ.

As used herein, the term "costimulatory domain" in the context of a chimeric antigen receptor refers to the intracellular signaling domain of a costimulatory molecule. The co-stimulatory molecules are cell surface molecules other than antigen receptors or Fc receptors, and the cell surface molecules provide the second signal required for the effective activation and functionality of T lymphocytes upon binding to an antigen. A non-limiting example of a costimulatory domain is the CD28 costimulatory domain.

The term "immune effector cell" or "effector cell" as used herein refers to a cell involved in an immune response, e.g., promoting an immune effector response. Examples of immune effector cells include T cells, e.g., α/β T cells and γ/δ T cells, B cells, natural killer (NK) cells, natural killer T (NKT) cells, mast cells, and myeloid-derived phagocytes.

The term "specific binding" herein means that an antigen-binding molecule (e.g., an antibody or a ligand of an antigen) specifically binds to an antigen and substantially identical antigens, generally with high affinity, but does not bind to unrelated antigens with high affinity. The affinity is generally reflected in an equilibrium dissociation constant (KD), where a relatively low KD indicates a relatively high affinity. In the case of antibodies, a high affinity generally refers to a KD of about 10⁻⁶ M or less, about 10⁻⁷ M or less, about 10⁻⁸ M or less, about 1 × 10⁻⁹ M or less, 1 × 10⁻¹⁰ M or less, 1 × 10⁻¹¹ M or less, or 1 × 10⁻¹² M or less. The KD is calculated as follows: KD = Kd/Ka, where Kd represents the dissociation rate, and Ka represents the association rate. The equilibrium dissociation constant KD can be measured by methods well known in the art, such as surface plasmon resonance (e.g., Biacore) or equilibrium dialysis. Illustratively, KD can be obtained by the method as described in the examples herein.

The term "antibody" herein is used in its broadest sense and refers to a polypeptide or a combination of polypeptides that comprises a sufficient sequence from an immunoglobulin heavy chain variable region and/or a sufficient sequence from an immunoglobulin light chain variable region to be capable of specifically binding to an antigen. "Antibody" herein encompasses various forms and various structures as long as they exhibit the desired antigen-binding activity, including intact antibodies and antigen-binding fragments thereof.

The term "antibody" herein includes a typical "four-chain antibody", which is an immunoglobulin consisting of two heavy chains (HCs) and two light chains (LCs). The heavy chain refers to a polypeptide chain consisting of, from the N-terminus to the C-terminus, a heavy chain variable region (VH), a heavy chain constant region CH1 domain, a hinge region (HR), a heavy chain constant region CH2 domain, a heavy chain constant region CH3 domain; moreover, when the full-length antibody is of IgE isoform, the heavy chain optionally further comprises a heavy chain constant region CH4 domain. The light chain is a polypeptide chain consisting of, from the N-terminus to the C-terminus, a light chain variable region (VL) and a light chain constant region (CL). The heavy chains are connected to each other and to the light chains through disulfide bonds to form a Y-shaped structure. The heavy chain constant regions of immunoglobulins differ in their amino acid composition and arrangement, and thus in their antigenicity. Accordingly, the "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. Light chains are divided into κ chains or λ chains according to differences in the constant regions. Each of the five classes of Ig may have a κ chain or a λ chain.

The terms "antigen-binding fragment" and "antibody fragment" herein are used interchangeably and refer to a fragment that does not have the entire structure of an intact antibody, but comprises only a portion of the intact antibody or a variant of the portion that retains the ability to bind to an antigen. The "antigen-binding fragment" or "antibody fragment" herein includes, but is not limited to, a Fab, a Fab', a Fab'-SH, a F(ab')₂, an scFv, and a VHH.

The term "scFv" (single-chain variable fragment) herein refers to a single polypeptide chain comprising VL and VH domains, wherein the VL and VH are linked through a linker (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Roseburg and Moore Ed., Springer-Verlag, New York, pp 269-315 (1994)). Such scFv molecules may have a general structure: NH₂-VL-linker-VH-COOH or NH₂-VH-linker-VL-COOH. An appropriate linker in the prior art includes GSTSGSGKPGSGEGSTKG and consists of GGGGS amino acid sequence repeats or a variant thereof. For example, a linker having the amino acid sequence (GGGGS)₄ can be used, and variants thereof can also be used (Holliger et al. (1993), Proc. Natl. Acad. Sci. USA 90: 6444-6448). Other linkers that can be used in the present disclosure are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol. In some cases, there may also be disulfide bonds between the VH and VL of the scFv, forming a disulfide-linked Fv (dsFv).

"Antibody" herein may be derived from any animal, including, but not limited to, human and non-human animals which may be selected from primates, mammals, rodents, and vertebrates, such as Camelidae species, *Lama glama*, *Lama guanicoe*, *Vicugna pacos*, sheep, rabbits, mice, rats, or *Chondrichthyes* (e.g., shark).

The term "humanized antibody" means an antibody obtained by grafting CDR sequences derived from another mammalian species, such as a mouse species, onto human framework sequences. To preserve the binding affinity, some residues in the backbone (referred to as FR) segments may be modified. The humanized antibodies or fragments thereof according to the present application can be prepared by techniques known to those skilled in the art.

The term "variable region" herein refers to a region of a heavy or light chain of an antibody involved in the binding of the antibody to an antigen. The "heavy chain variable region" is used interchangeably with "VH" and "HCVR", and the "light chain variable region" is used interchangeably with "VL" and "LCVR". The heavy and light chain variable domains (VH and VL, respectively) of natural antibodies generally have similar structures, each of which contains four conserved framework regions (FRs) and three hypervariable regions (HVRs). See, e.g., Kindt et al., Kuby Immunology, 6th ed., W. H. Freeman and Co., p.91 (2007). A single VH or VL domain may be sufficient to provide antigen-binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region as it is precisely complementary to an antigenic epitope in spatial structure, wherein the heavy chain variable region CDR may be abbreviated as HCDR and the light chain variable region CDR may be abbreviated as LCDR. The terms "framework region" and "FR" are used interchangeably and refer to those amino acid residues of an antibody heavy chain variable region or light chain variable region other than CDRs. Generally, a typical antibody variable region consists of 4 FRs and 3 CDRs in the following order: FR1-CDR1-FR2-CDR2-FR3-CDR3-FR4.

For further description of the CDRs, see Kabat et al., J. Biol. Chem., 252:6609-6616 (1977); Kabat et al., United States Department of Health and Human Services, Sequences of proteins of immunological interest (1991); Chothia et al., J. Mol. Biol. 196:901-917 (1987); Al-Lazikani B. et al., J. Mol. Biol., 273:927-948 (1997); MacCallum et al., J. Mol. Biol. 262:732-745 (1996); Abhinandan and Martin, Mol. Immunol., 45:3832-3839 (2008); Lefranc M.P. et al., Dev. Comp. Immunol., 27:55-77 (2003); and Honegger and Plückthun, J. Mol. Biol., 309:657-670 (2001). "CDR" herein may be labeled and defined in a manner well known in the art, including, but not limited to, Kabat numbering scheme; the tool sites used include, but are not limited to, abYsis site (www.abysis.org/abysis/sequence_input/key_annotation/key_annotation.cgi).

The term "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991).

As used herein, the terms "percent (%) sequence consistency" and "percent (%) sequence identity" are interchangeable, and refer to the percentage of identity between amino acid (or nucleotide) residues of a candidate sequence and amino acid (or nucleotide) residues of a reference sequence after aligning the sequences and introducing gaps, if necessary, to achieve maximum percent sequence identity (e.g., gaps may be introduced in one or both of the candidate sequence and the reference sequence for optimal alignment, and non-homologous sequences may be omitted for the purpose of comparison). Alignment may be carried out in a variety of ways well known to those skilled in the art for the purpose of determining percent sequence identity, for example, using publicly available computer software such as BLAST, ALIGN, or Megalign (DNASTAIi) software. Those skilled in the art can determine appropriate parameters for measuring alignment, including any algorithms required to achieve maximum alignment of the full length of the aligned sequences. For example, a reference sequence aligned for comparison with a candidate sequence may show that the candidate sequence exhibits 50% to 100% sequence identity over the full length of the candidate sequence or a selected portion of contiguous amino acid (or nucleotide) residues of the candidate sequence. The length of the candidate sequence aligned for the purpose of comparison may be, e.g., at least 30% (e.g., 30%, 40%, 50%, 60%, 70%, 80%, 90%, or 100%) of the length of the reference sequence. When a position in the candidate sequence is occupied by the same amino acid (or nucleotide) residue at the corresponding position in the reference sequence, then the molecules are identical at that position.

As used herein, "mutation" includes insertion mutation, deletion mutation and substitution mutation, and in some embodiments, the substitution mutation is preferably a conservative amino acid substitution.

As used herein, the term "conservative amino acid" generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
Illustratively, the following six groups are examples of amino acids that are considered to be conservative replacements of each other:
1) alanine (A), serine (S), and threonine (T);
2) aspartate (D) and glutamate (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

As used herein, "at most X mutations" means that the number of mutations may be selected from any natural number in the range of 0 to X.

As used herein, "at most 0-3 mutations" may be 3 mutations, 2 mutations, 1 mutation, or 0 mutations. As used herein, "respectively" of "the HCDR1-3 have at most 0-3 mutations, respectively", means that the mutation numbers per CDR in the HCDR1-3 are independent of each other and may each be independently selected from 0-3. Similarly, as used herein, "respectively" of "the LCDR1-3 have at most 0-3 mutations, respectively", means that the mutation numbers per CDR in the LCDR1-3 are independent of each other.

As used herein, the term "vector" is a composition of matter that comprises an isolated nucleic acid and can be used to deliver the isolated nucleic acid to the interior of a cell. A variety of vectors are known in the art, including but not limited to, linear polynucleotides, polynucleotides associated with ionic or amphiphilic compounds, plasmids, and viruses. Thus, the term "vector" includes an autonomously replicating plasmid or virus. The term should also be construed to include non-plasmid and non-viral compounds that facilitate the transfer of nucleic acids into cells, e.g., polylysine compounds and liposomes. Examples of viral vectors include, but are not limited to, adenoviral vectors, adeno-associated viral vectors, retroviral vectors, and the like.

As used herein, the terms "subject" and "patient" refer to an organism that receives administration of the nucleic acid molecule, the vector, the immune effector cell, the product, or the pharmaceutical composition described herein, wherein the "patient" also refers to an organism that receives treatment for a particular disease or disorder (e.g., cancer, an infectious disease, or an autoimmune disease) described herein. Examples of the patient include mammals, such as human, primate, pig, goat, rabbit, hamster, cat, dog, guinea pig, members of the bovine family (such as cattle, bison, buffalo, elk, yak, etc.), cattle, sheep, horse, bison, etc., that receive a treatment for a disease or disorder (e.g., a cell proliferative disorder, such as a cancer or an infectious disease or an autoimmune disease). Examples of subjects and subjects include, in addition to patients, healthy mammals, such as human, primate, pig, goat, rabbit, hamster, cat, dog, guinea pig, members of the bovine family (such as cattle, bison, buffalo, elk, yak, etc.), cattle, sheep, horse, bison, etc.

The term "cancer" herein refers to or describes a physiological condition in mammals that is typically characterized by unregulated cell growth. Included in this definition are benign and malignant cancers. The term "tumor" or "neoplasm" herein refers to all neoplastic cell growth and proliferation, whether malignant or benign, and all pre-cancerous and cancerous cells and tissues. The terms "cancer" and "tumor" are not mutually exclusive when referred to herein.

The term "B-cell malignancy" herein is a malignancy characterized by abnormal proliferation of B cells, and what is relatively common includes acute B-lymphocytic leukemia, non-Hodgkin's lymphoma, B-cell chronic lymphocytic leukemia, hairy cell leukemia, acute common B-lymphocytic leukemia, and the like.

In some cases, the term "acute lymphocytic leukemia (ALL)" herein is a malignant clonal disease that occurs when the genes of lymphoid progenitor cells are altered and cause uncontrolled cell proliferation. When the lymphoid progenitor cells undergo somatic mutation, uncontrolled proliferation occurs. This progressive clonal expansion can lead to the replacement of normal hematopoietic cells in the bone marrow by early lymphoid precursor cells, further infiltration of different organs, and finally to ALL. There are two types of ALL: acute B-lymphocytic leukemia (B-ALL) (derived from B cell line progenitor cells) and acute T-lymphocytic leukemia (T-ALL) (derived from T cell line progenitor cells), with B-ALL being the more common.

In some cases, the term "non-Hodgkin's lymphoma (NHL)" herein is a cancer that occurs in the lymphatic system. In NHL, white blood cells called lymphocytes grow abnormally and can form tumors throughout the body. NHL is the most common hematologic tumor in China and also in the world, and is generally classified into two types: aggressive and indolent NHL. Diffuse large B-cell lymphoma (DLBCL), the most common subtype of adult NHL, is an aggressive and highly heterogeneous disease. Follicular lymphoma (FL) is the most common indolent B-cell NHL and accounts for about 1/4 of all NHL. However, the FL grade 3B subgroup is rare.

In some cases, the term "chronic lymphocytic leukemia (CLL)" or small lymphocytic lymphoma (SLL) herein is a tumor of the same type that occurs at different sites. CLL tumor cells are mainly distributed in the blood and bone marrow, and SLL tumor cells are mainly distributed in the lymph nodes and spleen. CLL is a B-cell chronic lymphoproliferative disease (B-CLPD) in which mature B lymphocytes accumulate in peripheral blood, bone marrow, and lymphoid tissues and produce corresponding clinical symptoms. It is the most common leukemia and mainly affects B cells.

The term "autoimmune disease" herein refers to a condition resulting from an abnormal immune response attacking normal cells in the human body, which may occur in almost any part of the human body. Previous studies have shown that autoimmune diseases are highly associated with cancer, and suffering from autoimmune diseases will increase the risk or probability of certain cancers. Autoimmune diseases cause inflammation by various mechanisms and, in contrast, the risk depends on the autoimmune disease increasing chronic inflammation associated with cancer. Common autoimmune diseases associated with cancer include: celiac disease, inflammatory bowel disease (Crohn's disease and ulcerative colitis), multiple sclerosis, rheumatoid arthritis, and systemic lupus erythematosus. The autoimmune diseases described herein also include myositis, vasculitis, scleroderma, and the like. Herein, the terms "autoimmune disease" and "autoimmune-related disease" are used interchangeably.

In some cases, the term "systemic lupus erythematosus (SLE)" herein is a chronic autoimmune disease involving multiple organs, the main pathogenesis of which is autoimmune reaction caused by immune imbalance, and the most critical factor is the dysregulation of the activation of autoreactive B cells. Studies have shown that the senescent/autoimmune-related B cells are found to be the main cells for SLE autoantibody production. The B cells of SLE patients proliferate abnormally, produce a large amount of autoantibodies against self-components, bind to corresponding self-antigens in the body to form immune complexes, directly destroy cells, or cause acute and chronic inflammation and tissue necrosis under the action of complement effects, thereby causing multi-system damage to the body. The current treatment methods for SLE mainly include plasma replacement, injection of immunoglobulins, and administration of drugs targeting B cells, such as injection of monoclonal antibodies against B cells.

In some cases, the term "myositis" herein refers to inflammatory myopathy, which is a group of heterogeneous diseases characterized primarily by infiltration of skeletal muscle inflammatory cells and necrosis of muscle fibers. There are mainly two major classes. One is infectious myopathies with defined causes, such as viral myositis, parasitic myositis, and tropical myositis. The other is so-called idiopathic inflammatory myopathies (IIMs) with unknown but autoimmune-related causes, including polymyositis (PM), dermatomyositis (DM), and inclusion body myositis.

In some cases, the term "vasculitis" herein generally refers to systemic vasculitis, which is an autoimmune disease characterized by inflammation of blood vessels. Systemic vasculitis is a group of diseases involving various organs and varying degrees of clinical severity.

In some cases, the term "scleroderma" herein is a systemic autoimmune disease characterized by skin thickening, stiffness, and visceral fibrosis in a limited systemic form or in a diffuse systemic form. Depending on the area of skin involvement, it is divided into localized scleroderma, which involves skin symptoms only and does not involve the visceral system, or systemic sclerosis (SSc), which involves the visceral system.

As used herein, the term "treatment" refers to surgical or therapeutic treatment for the purpose of preventing or slowing (reducing) the progression of an undesirable physiological or pathological change, such as a cell proliferative disorder (e.g., a cancer or an infectious disease), in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

As used herein, the term "effective amount" refers to an amount of a therapeutic agent that is effective in preventing or alleviating the symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "pharmaceutical composition" herein means a combination of at least one drug and optionally a pharmaceutically acceptable carrier or excipient combined together to achieve a certain objective. In certain embodiments, the pharmaceutical composition includes temporally and/or spatially separated combinations, so long as they can act together to achieve the objective of the present application. For example, the components contained in the pharmaceutical composition (e.g., the CAR-NK cell according to the present application) may be administered to an individual as a whole or separately. When administered to an individual separately, the components contained in the pharmaceutical composition may be administered to the individual simultaneously or sequentially. The pharmaceutical composition according to the present application may comprise conventional components of cell culture to maintain the activity of the CAR-NK cells. The pharmaceutically acceptable carrier may also include water, an aqueous buffer solution, an isotonic salt solution such as PBS (phosphate-buffered saline), glucose, mannitol, dextrose, lactose, starch, magnesium stearate, cellulose, magnesium carbonate, 0.3% glycerol, hyaluronic acid, ethanol or a polyalkylene glycol such as polypropylene glycol, triglyceride, cell storage solution, colloidal dispersion system, macromolecular complex, nanocapsule, nanoparticle, microsphere, bead, etc. The pharmaceutical composition or pharmaceutical formulation according to the present application may be administered by any suitable route, for example, intravenous administration, intradermal, subcutaneous, and intramuscular injection, etc. The composition according to the present application may comprise a wetting agent, an emulsifier or a buffer substance as an additive.

The term "cryopreserved immune cells" herein refer to a cryopreserved substance obtained by resuspending immune cells in a cell cryopreservation solution and performing cryopreservation. In some cases, the cryopreserved immune cells should be a colorless or light yellow clear liquid without flocs after thawing.

The components in the term "cell storage solution" herein can effectively maintain cell morphology, prevent cell autolysis, ensure cell integrity, and ensure protein fixation and nucleic acid stability, and are used for preserving and transporting cells taken from a human body. The main components comprise a pH buffer, an osmotic pressure maintainer, a nucleic acid stabilizer, and the like. In some cases, the cell storage solution herein may be a cytoprotective solution or a cryoprotectant, which readily binds to water molecules in the solution to lower the freezing point and reduce the formation of ice crystals, and can reduce the concentration of electrolytes in the unfrozen solution through its molar concentration, thereby protecting cells from solute damage and ice crystal damage in the cells. The frozen cells retain their normal structures and functions. In some cases, the cell storage solution described herein comprises a compound electrolyte injection, a human serum albumin injection, a cryopreservation solution, or the like conventional in the art.

In some cases, the compound electrolyte injection described herein is a conventional compound electrolyte injection in the art, the main components of which include: sodium chloride, sodium gluconate, sodium acetate (C₂H₃NaO₂·3H₂O), potassium chloride, and magnesium chloride (MgCl₂·6H₂O), and the components can be optimized and adjusted if necessary; common products include compound electrolyte injections of Luoxin Pharmaceutical, Sihuan Pharmaceutical, Huaren Pharmaceutical, Kelun Pharmaceutical, etc., or Weidaiming^{®} compound electrolyte injection, Bomaili A, etc.

In some cases, the human serum albumin injection described herein is a conventional human serum albumin injection in the art, which comprises human serum albumin as a main component and comprises sodium octanoate and acetyl tryptophan as excipients. In some cases, the human serum albumin in the injection may have a concentration of 5%-25%, such as 5%, 10%, 20%, and 25%. Common products include human serum albumin injections of, e.g., CSL Behring, Octapharma, Grifols, Takeda Pharmaceuticals, Chengdu Rongsheng Pharmaceutical, Shenzhen Weiguang, Guangdong Weilun, etc.

In some cases, the cryopreservation solution described herein is a conventional commercial cryopreservation solution in the art and should contain cryoprotectants such as DMSO or glycerol, and common products include commercial cryopreservation solutions of Sigma-Aldrich, Sartorius, Gibco, ExCell, BioLife, etc.

### DETAILED DESCRIPTION

In a first aspect, the present application provides an immune effector cell comprising a chimeric antigen receptor targeting CD19, wherein the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3. In some embodiments, the HCDR1-3 have the sequences set forth in SEQ ID NOs: 5-7, respectively, and the LCDR1-3 have the sequences set forth in SEQ ID NOs: 8-10, respectively. In some embodiments, the HCDR1-3 have at most 0-3 mutations (e.g., 0, 1, 2, and 3 mutations) compared with the corresponding CDRs set forth in SEQ ID NOs: 5-7. In some embodiments, the LCDR1-3 have at most 0-3 mutations (e.g., 0, 1, 2, and 3 mutations) compared with the corresponding CDRs set forth in SEQ ID NOs: 8-10.

In some embodiments, (1) the VH has the sequence set forth in SEQ ID NO: 20, and the VL has the sequence set forth in SEQ ID NO: 17; or (2) the VH has a sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 25 mutations (e.g., at most 20 mutations, at most 15 mutations, at most 10 mutations, at most 5 mutations, at most 4 mutations, at most 3 mutations, at most 2 mutations, or 1 mutation) compared with the corresponding VH in group (1), and the VL has a sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 20 mutations (e.g., at most 15 mutations, at most 10 mutations, at most 5 mutations, at most 4 mutations, at most 3 mutations, at most 2 mutations, or 1 mutation) compared with the corresponding VL in group (1).

In some embodiments, the chimeric antigen receptor has the sequence set forth in SEQ ID NO: 23, or has a sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 120 amino acid mutations (e.g., at most 110 mutations, at most 100 mutations, at most 90 mutations, at most 80 mutations, at most 70 mutations, at most 60 mutations, at most 50 mutations, at most 45 mutations, at most 40 mutations, at most 35 mutations, at most 30 mutations, at most 25 mutations, at most 20 mutations, at most 15 mutations, at most 10 mutations, at most 5 mutations, at most 4 mutations, at most 3 mutations, at most 2 mutations, or 1 mutation) compared with SEQ ID NO: 23.

In a second aspect, the present application provides a pharmaceutical composition comprising the immune effector cell according to the first aspect and a pharmaceutically acceptable carrier.

In some embodiments, the pharmaceutically acceptable carrier includes water, buffer, glucose, mannitol, dextrose, lactose, starch, magnesium stearate, cellulose, magnesium carbonate, 0.3% glycerol, hyaluronic acid, ethanol, a polyalkylene glycol such as polypropylene glycol, triglyceride, cell storage solution, colloidal dispersion system, macromolecular complex, nanocapsule, nanoparticle, microsphere, and bead.

In some embodiments, the pharmaceutical composition is cryopreserved immune cells.

In some embodiments, the pharmaceutically acceptable carrier is a cell storage solution; in some preferred embodiments, components of the cell storage solution include a compound electrolyte injection and a human serum albumin injection and/or cryopreservation solution.

In some preferred embodiments, the compound electrolyte injection is a conventional compound electrolyte injection in the art, the main components of which include: sodium chloride, sodium gluconate, sodium acetate (C₂H₃NaO₂·3H₂O), potassium chloride, and magnesium chloride (MgCl₂·6H₂O), and the components can be optimized and adjusted if necessary; common products include compound electrolyte injections of Luoxin Pharmaceutical, Sihuan Pharmaceutical, Huaren Pharmaceutical, Kelun Pharmaceutical, etc., or Weidaiming^{®} compound electrolyte injection, Bomaili A, etc.

In some cases, the human serum albumin injection described herein is a conventional human serum albumin injection in the art, which comprises human serum albumin as a main component and comprises sodium octanoate and acetyl tryptophan as excipients. In some cases, the human serum albumin in the injection may have a concentration of 5%-25%, such as 5%, 10%, 20%, and 25%. Common products include human serum albumin injections of, e.g., CSL Behring, Octapharma, Grifols, Takeda Pharmaceuticals, Chengdu Rongsheng Pharmaceutical, Shenzhen Weiguang, Guangdong Weilun, etc.

In some preferred embodiments, the cryopreservation solution is a conventional commercial cryopreservation solution in the art and should contain cryoprotectants such as DMSO or glycerol, such as commercial cryopreservation solutions of Sigma-Aldrich, Sartorius, Gibco, ExCell, BioLife, etc.

In some embodiments, the immune effector cell cryopreservation density in the cryopreserved immune cells is no less than 1 × 10⁵ viable cells/mL; in some preferred embodiments, the immune effector cell cryopreservation density in the cryopreserved immune cells is no less than 1 × 10⁶ viable cells/mL.

In some embodiments, the pharmaceutical composition is an injection.

In some preferred embodiments, the pharmaceutical composition is administered by intravenous drip infusion.

In some embodiments, the proportion of CD3⁺ cells in the pharmaceutical composition is no more than 5%.

In some preferred embodiments, the proportion of CD3⁺ cells in the immune effector cells is no more than 3%.

In some more preferred embodiments, the proportion of CD3⁺ cells in the immune effector cells is no more than 1%.

In a third aspect, the present application provides the immune effector cell according to the first aspect or the pharmaceutical composition according to the second aspect for use in preventing or treating a disease.

In a fourth aspect, the present application provides use of the immune effector cell according to the first aspect or the pharmaceutical composition according to the second aspect in the preparation of a medicament for treating a disease.

In a fifth aspect, the present application provides a method for preventing or treating a disease, which comprises administering to a subject in need an effective amount of the immune effector cell according to the first aspect or the pharmaceutical composition according to the second aspect.

In some embodiments, the disease is a B cell-driven disease.

In some preferred embodiments, the disease is a CD19-positive B cell-driven disease.

In some embodiments, the disease is a tumor or cancer.

In some embodiments, the tumor or cancer is a CD19-related tumor or cancer. In some embodiments, the tumor or cancer is a B-cell malignancy. In some embodiments, the tumor or cancer is a recurrent or refractory CD19-positive B-cell malignancy. In some embodiments, the tumor or cancer is acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

In some embodiments, the CD19-related tumor or cancer means that a tumor cell or cancer cell surface expresses CD19. In some embodiments, the tumor is a tumor highly expressing CD19 (CD19+). In some embodiments, the tumor that highly expressing CD19 (CD19+) refers to a tumor cell population in which at least 60% of the tumor cells express CD19. In some embodiments, the tumor that highly expressing CD19 (CD19+) refers to a tumor cell population in which at least 70% of the tumor cells express CD19. In some embodiments, the tumor that highly expressing CD19 (CD19+) refers to a tumor cell population in which at least 80% of the tumor cells express CD19. In some embodiments, the tumor that highly expressing CD19 (CD19+) refers to a tumor cell population in which at least 90% of the tumor cells express CD19. In some embodiments, the tumor that highly expressing CD19 (CD19+) refers to a tumor cell population in which at least 95% of the tumor cells express CD19. In some embodiments, the tumor that highly expressing CD19 (CD19+) refers to a tumor cell population in which at least 98% of the tumor cells express CD19. In some embodiments, the tumor that highly expressing CD19 (CD19+) refers to a tumor cell population in which at least 99% of the tumor cells express CD19.

In some embodiments, the prevention or treatment comprises administering to a subject in need the immune cell according to the first aspect or the pharmaceutical composition according to the second aspect.

In some embodiments, the subject has the tumor or cancer described herein.

In some embodiments, the subject has previously received at least second-line treatment.

In some embodiments, the subject has at least 1 measurable lesion according to Lugano 2014 Response Evaluation Criteria in Lymphoma (Cheson 2014).

In some embodiments, the disease is an autoimmune disease.

In some embodiments, the autoimmune disease includes systemic lupus erythematosus (SLE), inflammatory bowel disease (IBD), multiple sclerosis (MS), rheumatoid arthritis (RA), type I diabetes (T1DM), Sjogren's syndrome, myositis, vasculitis, scleroderma, and the like.

In some embodiments, the subject has the autoimmune disease described herein. In a preferred embodiment, the autoimmune disease is systemic lupus erythematosus, myositis, vasculitis, and scleroderma. In a more preferred embodiment, the autoimmune disease is moderate to severe active systemic lupus erythematosus, refractory idiopathic myositis, refractory granulomatosis with polyangiitis, or systemic sclerosis.

In some embodiments, the subject is diagnosed with SLE at least 24 weeks before screening.

In some embodiments, the 2019 European Alliance of Associations for Rheumatology (EULAR)/American College of Rheumatology (ACR) classification criteria for SLE are met at screening.

In some embodiments, administration of the pharmaceutical composition is a single administration or multiple administration.

In some embodiments, the administration is performed once a week.

In some embodiments, the single administration is 1 administration.

In some embodiments, the multiple administration is 2-6 administrations; in some preferred embodiments, the multiple administration is 2, 3, 4, 5 and 6 administrations.

In some embodiments, the pharmaceutical composition is administered at 1 × 10⁴ cells/Kg to 1 × 10⁹ cells/Kg each time.

In some preferred embodiments, the pharmaceutical composition is administered at 1 × 10⁴ cells/Kg to 1 × 10⁵ cells/Kg each time; in some preferred embodiments, the pharmaceutical composition is administered at 1 × 10⁵ cells/Kg to 1 × 10⁶ cells/Kg each time; in some preferred embodiments, the pharmaceutical composition is administered at 1 × 10⁶ cells/Kg to 1 × 10⁷ cells/Kg each time; in some preferred embodiments, the pharmaceutical composition is administered at 1 × 10⁷ cells/Kg to 1 × 10⁸ cells/Kg each time; in some preferred embodiments, the pharmaceutical composition is administered at 1 × 10⁸ cells/Kg to 1 × 10⁹ cells/Kg each time.

In some preferred embodiments, the pharmaceutical composition is administered at 1 × 10⁶ cells/Kg to 9 × 10⁸ cells/Kg each time, for example, at 1 × 10⁷ cells/Kg to 9 × 10⁸ cells/Kg.

In some preferred embodiments, the pharmaceutical composition is administered at 1 × 10⁶ cells/Kg to 9 × 10⁶ cells/Kg, 1 × 10⁷ cells/Kg to 9 × 10⁷ cells/Kg, or 1 × 10⁸ cells/Kg to 9 × 10⁸ cells/Kg each time.

In some more preferred embodiments, the pharmaceutical composition is administered at 1 × 10⁶ cells/Kg, 2 × 10⁶ cells/Kg, 3 × 10⁶ cells/Kg, 4 × 10⁶ cells/Kg, 5 × 10⁶ cells/Kg, 6 × 10⁶ cells/Kg, 7 × 10⁶ cells/Kg, 8 × 10⁶ cells/Kg, 9 × 10⁶ cells/Kg, 1 × 10⁷ cells/Kg, 2 × 10⁷ cells/Kg, 3 × 10⁷ cells/Kg, 4 × 10⁷ cells/Kg, 5 × 10⁷ cells/Kg, 6 × 10⁷ cells/Kg, 7 × 10⁷ cells/Kg, 8 × 10⁷ cells/Kg, 9 × 10⁷ cells/Kg, 1 × 10⁸ cells/Kg, 2 × 10⁸ cells/Kg, 3 × 10⁸ cells/Kg, 4 × 10⁸ cells/Kg, 5 × 10⁸ cells/Kg, 6 × 10⁸ cells/Kg, 7 × 10⁸ cells/Kg, 8 × 10⁸ cells/Kg, or 9 × 10⁸ cells/Kg each time.

It should be understood that the above detailed description is provided solely to enable those skilled in the art to understand the contents of the present application more clearly, and is not intended to be limiting in any way. Those skilled in the art are able to make various modifications and changes to the described embodiments.

### EXAMPLES

The present application will be further described with reference to specific examples, and the advantages and features of the present application will become more apparent with the description. Experimental procedures without specified conditions in the examples are conducted according to conventional conditions or conditions recommended by the manufacturers. Reagents or instruments without specified manufacturers used herein are conventional products that are commercially available.

The examples of the present application are exemplary only, and do not limit the scope of the present application in any way. It will be understood by those skilled in the art that various modifications or substitutions may be made to the technical solutions of the present application in form and details without departing from the spirit and scope of the present application, and that these modifications and substitutions shall fall within the protection scope of the present application.

### Materials and methods

### SPR detection method

The anti-human CD19 antibody was captured using a Protein A chip (GE Healthcare; 29-127-558). The sample and running buffer was HBS-EP+ (10 mM HEPES, 150 mM NaCl, 3 mM EDTA, 0.05% surfactant P20) (GE Healthcare; BR-1006-69). The flow cell was set at 25 °C. The sample block was set at 16 °C. Both were pretreated with the running buffer. In each cycle, first, the antibody to be tested was captured using the Protein A chip, and then a single concentration of CD19 antigen protein was injected. The association and dissociation processes of the antibody with the antigen protein were recorded, and finally, the chip was regenerated using Glycine pH 1.5 (GE Healthcare; BR-1003-54). The association was determined by injecting different concentrations of recombinant human CD19-His in the solution and maintaining for 240 s, wherein the flow rate was 30 µL/min, and the protein was diluted in a 1:1 dilution ratio from 200 nM (see detailed results for actual concentrations tested) to obtain 5 concentrations in total. The dissociation phase was monitored for up to 600 s and triggered by switching from the sample solution to the running buffer. The surface was regenerated by washing with 10 mM glycine solution (pH 1.5) at a flow rate of 30 µL/min for 30 s. The difference in bulk refractive index was corrected by subtracting the responses obtained from the goat anti-human Fc surface. Blank injections were also subtracted (= double reference). The Langmuir 1:1 model was used to calculate the apparent KD and other kinetic parameters.

### Virus packaging

The day before virus packaging, 293T cells (purchased from ATCC) were digested with trypsin and inoculated onto 10 cm culture dishes at a density of 1E7 cells per culture dish. When the cells were transfected, the packaging plasmids and the target plasmids were mixed and then added to an α-MEM culture medium, and the FuGENE^{®} HD transfection reagent (Promega, E2311) was added to another centrifuge tube containing the α-MEM culture medium. The diluted transfection reagent was added dropwise above the diluted plasmid, and the mixture was well mixed, and left to stand at room temperature for 15 min. Finally, the mixture of plasmid and the transfection reagent was added to a 10 cm culture dish, gently shaken 10 times to mix well, and put into an incubator. Three days after the cell transfection, the viruses were harvested, and 10 mL of virus-containing culture supernatant was transferred to a 50 mL centrifuge tube and centrifuged at 1250 rpm at 4 °C for 5 min to remove dead 293T cells. The virus-containing supernatant was filtered, concentrated using the Retro-X Concentrator reagent (Clontech, 631455), and after packaging, stored at -80 °C for later use.

### NK cell purification and activation

NK cells were separated from monocytes. The purification was divided into two steps, namely removing CD3⁺ cells in the first step, and enriching CD56⁺ cells in the second step. Cell washing, CD3 magnetic bead incubation and resuspension were performed using Sepax, followed by removal of the CD3⁺ cells with CliniMACS. The CD3⁻ cell product after removal of CD3⁺ cells was subjected to washing, CD56 magnetic bead incubation and resuspension using Sepax again, and CD56⁺ cells were enriched with CliniMACS. The enriched CD56⁺ cells were washed with Sepax and exchanged into a complete medium. K562 feeder cells were added, and the mixture was transferred to a 37 °C incubator for 5-day activation.

### Virus transduction

On day 1, RetroNectin reagent was added to 24-well plates at 500 µL/well and coated at 4 °C overnight. On day 2, the upper-layer RetroNectin was discarded, and the plate was washed with PBS. Packaged retrovirus was added and centrifuged at 4-8 °C at 2000 g for 60 min. The virus supernatant was discarded. The NK cells activated for 5 days were added to 24-well plates at 3E5 cells/well, centrifuged at room temperature at 400 g for 5 min, and cultured in a 37 °C incubator (37 °C, 5% CO₂). On day 3, the NK cells were transferred to 6-well plates without tissue culture treatment and cultured. On day 6, CAR expression was detected to ensure the CAR positive rate in the range of 60% to 80% for NK cells used for *in vitro* or *in vivo* killing.

### Determination of in vitro tumor cell killing rate

Unless otherwise specified, the target cells referred to in the following examples are stably transfected with a luciferase gene to express luciferase. The luciferase reporter gene assay reagent was used for determining the fluorescence intensity and reflecting the cell viability and the killing effect of NK cells. The experimental well killing rate calculation formula is as follows: killing rate = (target cell well reading - experimental well reading)/target cell well reading × 100%.

### Example 1. Preparation and Screening of Humanized Chimeric Antigen Receptor Targeting CD19

### 1.1 Preparation of CD19 antibodies

Hybridoma-positive clone antibodies were prepared using human CD19 protein (NCBI: NP_001761.3) as an immunogen by conventional antibody preparation and screening methods in the art. The sequences of the heavy and light chain variable regions of the preferred anti-CD19 antibody Mab01 and the CDRs (Kabat numbering scheme) thereof, as well as the sequence of the positive control FMC63, were determined and are shown in Tables 1 and 2. The SPR detection results are shown in Table 3. Mab01 had good affinity for human CD19 protein.

**Table 1. Sequences of antibodies**

| Sequence name | SEQ ID NO: | Sequence |
|---|---|---|
| Mab01-VH | 1 | QVQLQQPGAELVVPGTSVKLSCKASGYTFTNYWMHWVKQRPGQGLE WIGEIDPSDNYANYNQEFQGKATLTVDKSSSTAYMQLSSLTSDDSAVY YCARHDGYFGALDYWGQGTSVTVSS |
| Mab01-VL | 2 | EIVLTQSPTTMAASPGEKITITCSASSSISSNYLHWYQQKPGFSPKFLIYR TSNLASGVPARFSGSGSGTSYSLTIGTMEAEDVATYYCQQASSIPRMFT FGSGTKLEIK |
| FMC63-VH | 3 | EVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCAK HYYYGGSYAMDYWGQGTSVTVSS |
| FMC63-VL | 4 | DIQMTQTTSSLSASLGDRVTISCRASQDISKYLNWYQQKPDGTVKLLIY HTSRLHSGVPSRFSGSGSGTDYSLTISNLEQEDIATYFCQQGNTLPYTFG GGTKLEIT |

**Table 2. CDRs corresponding to antibodies**

| Sequence name | CDR1 | CDR2 | CDR3 |
|---|---|---|---|
| Mab01-VH | NYWMH (SEQ ID NO:5) | EIDPSDNYANYNQEFQG (SEQ ID NO:6) | HDGYFGALDY (SEQ ID NO:7) |
| Mab01-VL | SASSSISSNYLH (SEQ ID NO:8) | RTSNLAS (SEQ ID NO:9) | QQASSIPRMFT (SEQ ID NO:10) |
| FMC63-VH | DYGVS (SEQ ID NO:11) | VIWGSETTYYNSALKS (SEQ ID NO:12) | HYYYGGSYAMDY (SEQ ID NO:13) |
| FMC63-VL | RASQDISKYLN (SEQ ID NO:14) | HTSRLHS (SEQ ID NO:15) | QQGNTLPYT (SEQ ID NO:16) |

**Table 3. Assay on affinity of chimeric antibodies for human CD19 by SPR (Biacore)**

| Antibody name | Ka (1/Ms) | Kd (1/s) | KD (M) |
|---|---|---|---|
| Mab01 | 3.27E+05 | 3.75E-04 | 1.15E-09 |
| FMC63 | 1.82E+05 | 3.33E-04 | 1.83E-09 |

### 1.2 Humanization of antibodies

The amino acid residues of the CDRs of the antibodies obtained by humanizing the anti-CD19 antibody Mab01 obtained by the above screening were determined by the Kabat numbering scheme. The sequence information of the humanized VH and VL is shown in Table 4, and the resulting humanized antibody variable region combinations are shown in Table 5. The binding ability of the chimeric antibodies to human CD19 protein was detected by SPR (Biacore), as detailed in Table 6 and Table 7. The obtained humanized antibodies had strong binding ability to human CD19 protein. The positive control FMC63 was subjected to humanization design in the same way. The humanized antibody hFMC63 was constructed and expressed, and the binding ability of the antibody to human CD19 protein was detected by SPR (Biacore). The sequence information of hFMC63 and its binding ability to human CD19 protein are shown in Tables 4 and 7, respectively. The results show that hFMC63 had a relatively strong binding ability to human CD19.

**Table 4. Sequence information of humanized antibodies of Mab01 and FMC63**

| Sequence name | SEQ ID NO: | Sequence |
|---|---|---|
| Hab01-VL1 | 17 | EIVLTQSPATLSLSPGERATLSCSASSSISSNYLHWYQQKPGQAPRFLIY RTSNLASGIPARFSGSGSGTDFTLTISSLEPEDFAVYYCQQASSIPRMFTF GQGTKLEIK |
| Hab01-VL3 | 18 | EIVLTQSPDFQSVTPKEKVTITCSASSSISSNYLHWYQQKPDQSPKFLIYR TSNLASGVPSRFSGSGSGTDFTLTINSLEAEDAATYYCQQASSIPRMFTF GQGTKLEIK |
| Hab01-VH3 | 19 | EVQLVQSGAEVKKPGASVKVSCKASGYTFTNYWMHWVRQRPGQGLE WMGEIDPSDNYANYNQEFQGRVTITVDKSASTAYMELSSLRSEDTAVY YCARHDGYFGALDYWGQGTTVTVSS |
| Hab01-VH4 | 20 | EVQLQQSGAEVKKPGASVKVSCKASGYTFTNYWMHWVRQRPGQGLE WMGEIDPSDNYANYNQEFQGRVTITVDKSASTAYMELSSLRSEDTAVY YCARHDGYFGALDYWGQGTTVTVSS |
| hFMC63-VL1 | 21 | DIQMTQSPSSLSASVGDRVTITCRASQDISKYLNWYQQKPGGAVKLLIY HTSRLHSGVPSRFSGSGSGTDYTLTISSLQPEDFATYYCQQGNTLPYTFG GGTKVEIK |
| hFMC63-VH1 | 22 | EVTLKESGPVLVKPTETLTLTCTVSGFSLSDYGVSWIRQPPGKALEWLA VIWGSETTYYNSALKSRLTISKDTSKSQVVLTMTNMDPVDTATYYCAK HYYYGGSYAMDYWGQGTTVTVSS |

**Table 5. Mab01 humanized antibody variable region combinations**

| Fv | Hab01-VH3 | Hab01-VH4 |
|---|---|---|
| Hab01-VL1 | / | Hab01-4 |
| Hab01-VL3 | Hab01-11 | / |

**Table 6. Assay on affinity of Mab01 humanized antibody for human CD19 by SPR (Biacore)**

| Antibody name | Ka (1/Ms) | Kd (1/s) | KD (M) |
|---|---|---|---|
| Hab01-4 | 9.11E+04 | 1.86E-08 | 2.05E-13 |
| Hab01-11 | 1.18E+05 | 1.04E-06 | 8.80E-12 |

**Table 7. Assay on affinity of FMC63 humanized antibody for human CD19 by SPR (Biacore)**

| Antibody name | Ka (1/Ms) | Kd (1/s) | KD (M) |
|---|---|---|---|
| hFMC63 | 9.24E+04 | 1.80E-06 | 1.95E-11 |
| FMC63 | 9.53E+04 | 1.17E-04 | 1.23E-09 |

### Example 2. In Vitro Validation and In Vivo Validation of Killing Activity of Humanized Chimeric Antigen Receptor Targeting CD19

### 2.1 Preparation of CD19 CAR-NK cells

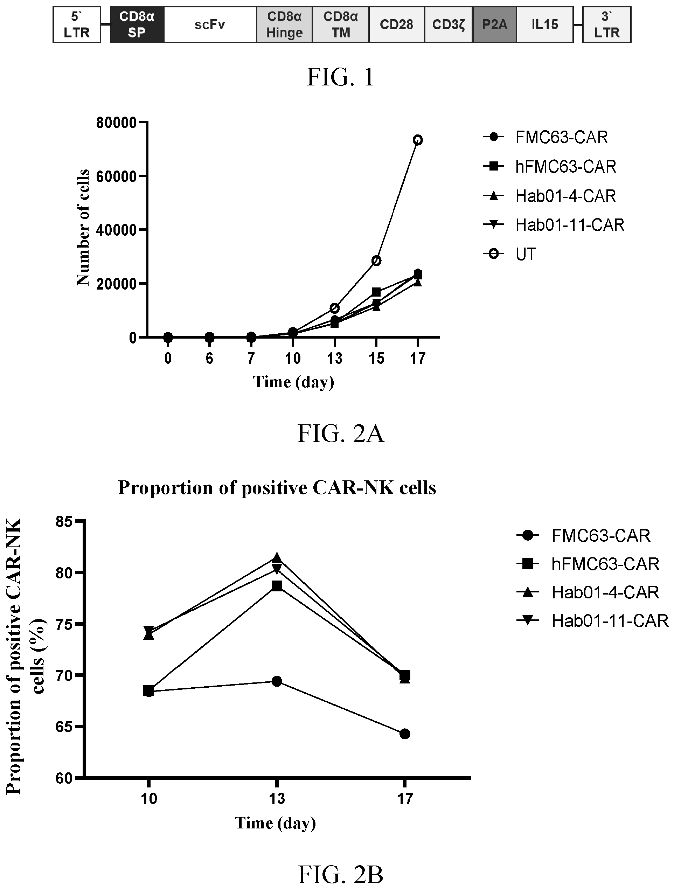

According to the structural schematic figure of the chimeric antigen shown in FIG. 1, the humanized antibodies Hab01-4, Hab01-11, hFMC63, and FMC63 prepared in Example 1 were used to construct a chimeric antigen receptor targeting CD19 (see Table 8 for specific sequence information), and the nucleic acid molecule encoding the chimeric antigen receptor was cloned into a conventional retroviral vector in the art. CD19 CAR NK cells were prepared using the methods of virus packaging, NK cell purification and activation, and virus transduction described in the Materials and Methods section above, and the expansion of NK cells after transfection and the CAR positive rate were detected. The results are shown in FIGs. 2A-2B for details.

### 2.2 In vitro killing activity of CD19 CAR-NK cells against Raji cells (Burkitt lymphoma cell line)

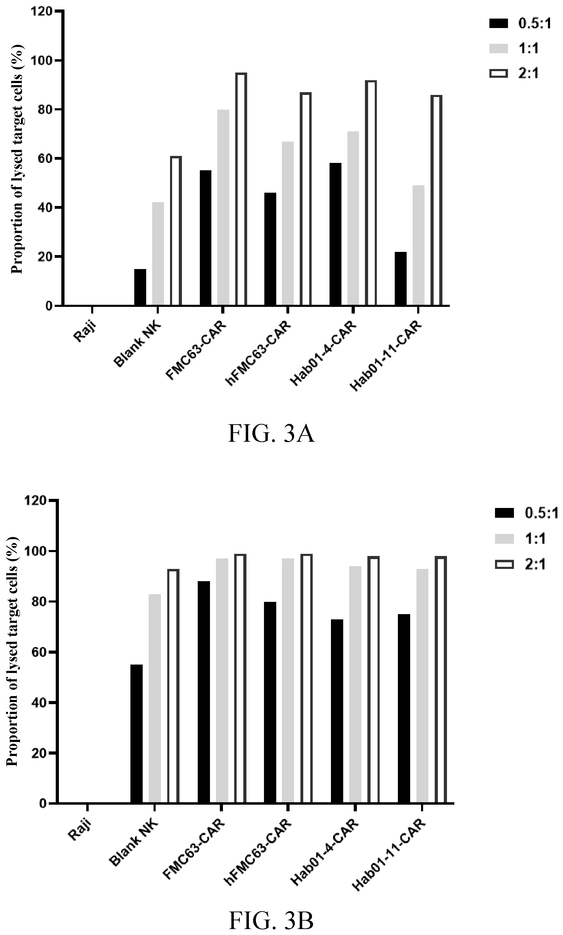

Before the experiment for detecting the killing activity on the target cells, the cryopreserved CD19 CAR-NK cells were thawed, and the CD19 CAR-NK cells with the same number were respectively taken to be mixed with the target cells Raji at the beginning of thawing (0 h) and 24 h after thawing. The *in vitro* killing effect of the NK cells on the target cells under different effector-to-target ratios (Raji: 1E6 cells/mL) was detected, and the results are shown in FIGs. 3A-3B. FMC63-CAR, hFMC63-CAR, Hab01-4-CAR and Hab01-11-CAR which were just thawed (FIG. 3A) and 24 h after thawing (FIG. 3B) had strong killing effect on Raji cells; in contrast, after 24 h of thawing, the killing effect of CD19 CAR-NK cells was better.

### 2.3 Evaluation of in vivo efficacy of CD19 CAR-NK cells in Nalm6 (human acute lymphocytic leukemia (ALL) cell line) xenograft mouse model

A Nalm6 xenograft mouse model was constructed using conventional methods in the art to evaluate the tumor inhibition of FMC63-CAR, hFMC63-CAR, Hab01-4-CAR, and Hab01-11-CAR.

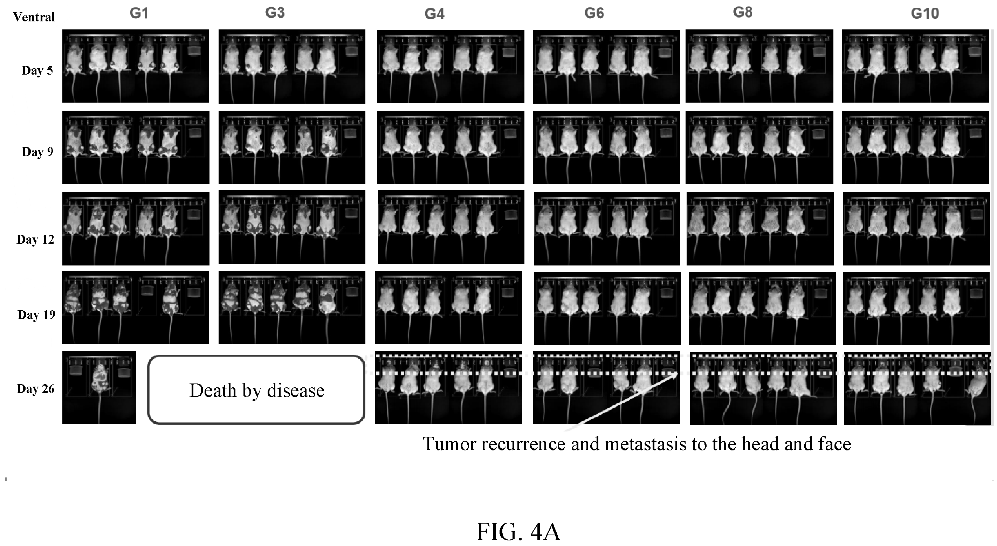

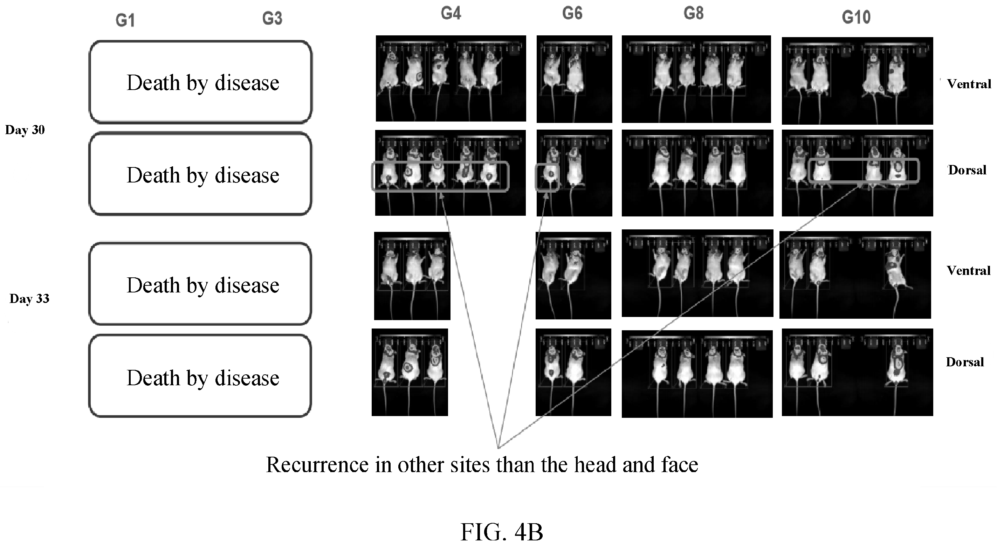

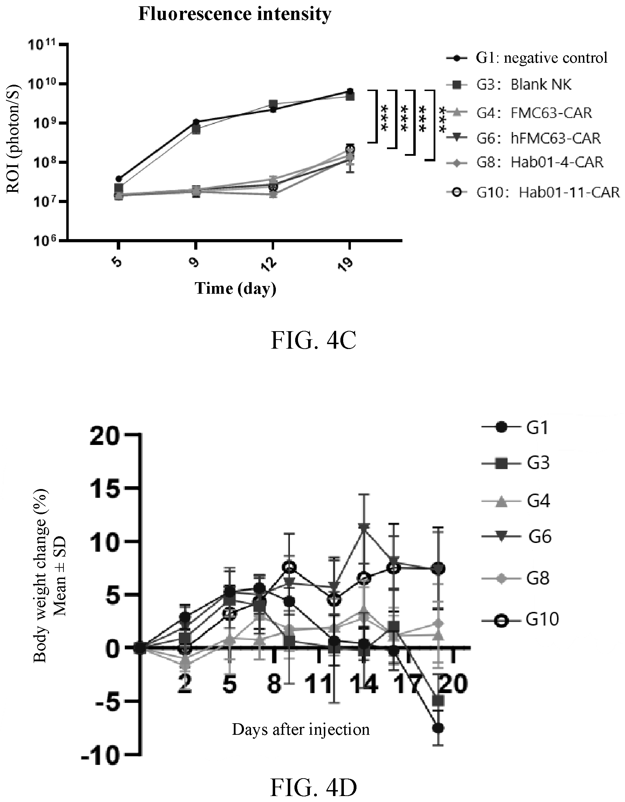

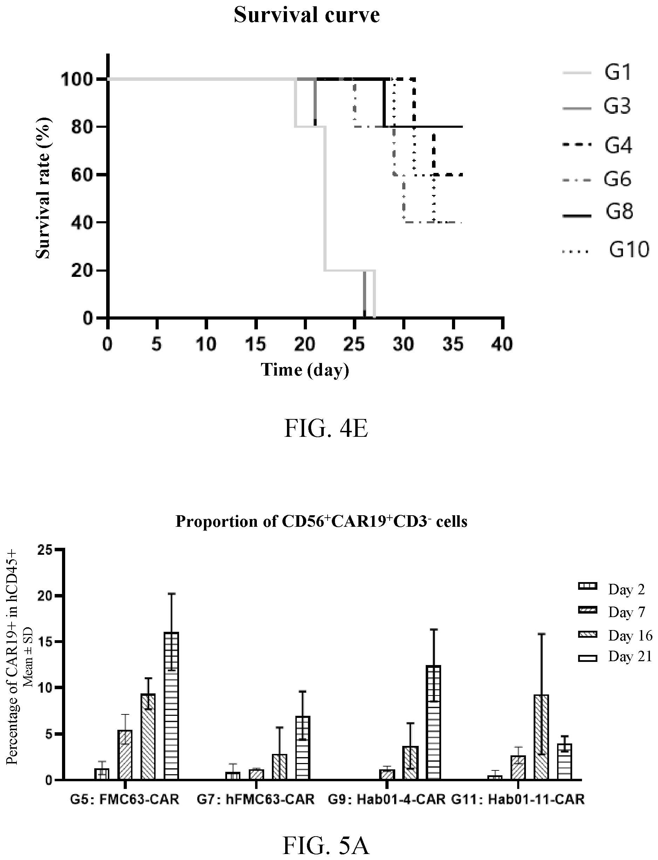

Specifically, NOG mice were weighed, randomly grouped (see Table 9 for the grouping protocol), injected with human acute lymphocytic leukemia (ALL) cell line Nalm6 (carrying the luciferase gene) at a dose of 2.5E6 cells/mouse, and intravenously injected with a single dose of CD19 CAR-NK cells (4E6 CD19 CAR-NK cells/mouse) 2 days later. The time point for injection of CD19 CAR-NK cells was defined as day 0. The *in vivo* tumor development (fluorescence imaging), mouse body weight and survival status of the mice in groups G1, G3, G4, G6, G8, and G10 were continuously observed. The ratio of CAR19⁺CD56⁺ to CAR19⁻CD56⁺ was determined by taking peripheral blood from the mice in groups G2, G5, G7, G9 and G11 on days 0, 7, 16 and 21, and the blood routine examination and blood biochemistry indexes of the peripheral blood were determined on days 0, 7, 14 and 28. The results are shown in FIG. 4A to FIG. 4E, FIG. 5A to FIG. 5B, and FIG. 6.

As shown in FIG. 4A to FIG. 4E, the tumor signals of the negative control groups (G1 and G3) continued to increase after tumor bearing, and the 4 CD19 CAR-NK treatment groups (G4, G6, G8 and G10) all showed significant antitumor efficacy, which significantly prolonged the survival time of the mice. In the 4 CD19 CAR-NK treatment groups, the Hab01-4-CAR-NK treatment group (group G8) had the best therapeutic effect. Although group G8 had recurrence of tumor metastasis to the head and face on day 26 as the other treatment groups, it was found that group G8 had no recurrence at other sites during days 30-33 by dorsal and ventral imaging observations, while the other treatment groups had recurrence at other sites than the head and face.

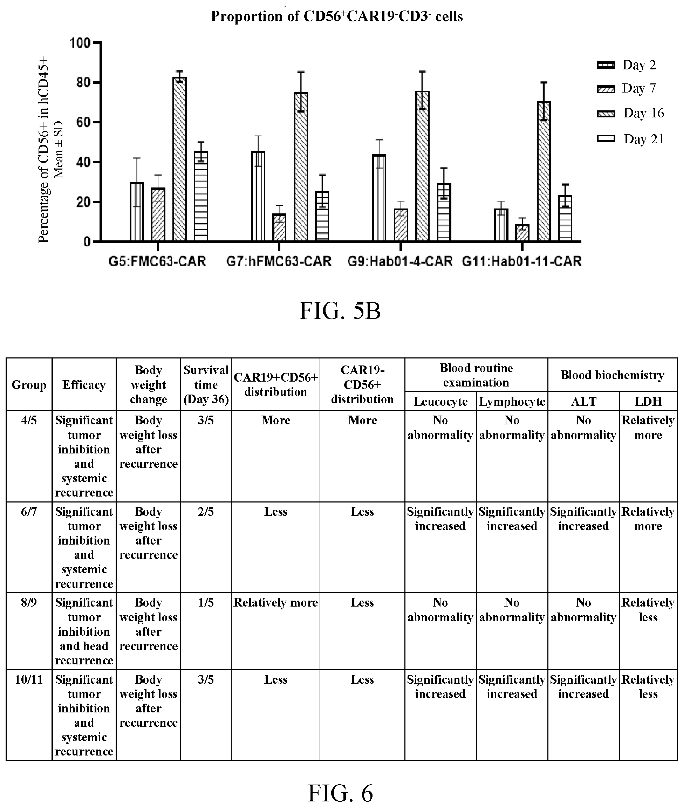

Meanwhile, from the perspective of survival curve, the survival rate of the mice in group G8 was 80% (day 36), which was superior to that of the other treatment groups. In terms of body weight change, the 4 CD19 CAR-NK treatment group had well-conditioned mice without treatment-related toxicity such as body weight loss before day 19. Body weight loss began to appear on day 19, while fluorescence signals were detected at the mandible. The body weight loss of the mice was caused by tumor recurrence. As shown in FIG. 5A to FIG. 5B, in terms of the CD19 CAR⁺CD56⁺ proportion, group G5 > group G9 > group G11 > group G7; in terms of the CD19 CAR⁻CD56⁺ proportion, group G11 < group G9 = group G7 < group G5; in the G5, G7, G9, and G11 groups using humanized antibodies, group G9 had a higher relative number of the CAR⁺ NK cells in the peripheral blood mononuclear cells. The blood routine examination and blood biochemistry indexes are detailed in FIG. 6. In group G9, the levels of alanine transaminase (ALT) and lactate dehydrogenase (LDH) were lower relative to the other CD19 CAR-NK treatment groups, indicating a greater safety overall.

**Table 8. Sequence information of chimeric antigen receptor targeting CD19**

| Name | SEQ ID NO: | Sequence information |
|---|---|---|
| Hab01-4-CAR | 23 | MALPVTALLLPLALLLHAARPLEEIVLTQSPATLSLSPGERATLSCSASSSISSN YLHWYQQKPGQAPRFLIYRTSNLASGIPARFSGSGSGTDFTLTISSLEPEDFAV YYCQQASSIPRMFTFGQGTKLEIKGSTSGSGKPGSGEGSTKGEVQLQQSGAEV KKPGASVKVSCKASGYTFTNYWMHWVRQRPGQGLEWMGEIDPSDNYANY NQEFQGRVTITVDKSASTAYMELSSLRSEDTAVYYCARHDGYFGALDYWGQ GTTVTVSSGSFVPVFLPAKRTTTPAPRPPTPAPTIASQPLSLRPEACRPAAGGA VHTRGLDFACDIYIWAPLAGTCGVLLLSLVITLYCRSKRSRLLHSDYMNMTPR RPGPTRKHYQPYAPPRDFAAYRSRVKFSRSADAPAYQQGQNQLYNELNLGR REEYDVLDKRRGRDPEMGGKPRRKNPQEGLYNELQKDKMAEAYSEIGMKG ERRRGKGHDGLYQGLSTATKDTYDALHMQALPPRGSGATNFSLLKQAGDVE ENPGPMRISKPHLRSISIQCYLCLLLNSHFLTEAGIHVFILGCFSAGLPKTEANW VNVISDLKKIEDLIQSMHIDATLYTESDVHPSCKVTAMKCFLLELQVISLESGD ASIHDTVENLIILANNSLSSNGNVTESGCKECEELEEKNIKEFLQSFVHIVQMFI |
| | | NTS |
| Hab01-11-CAR | 24 | MALPVTALLLPLALLLHAARPLEEIVLTQSPDFQSVTPKEKVTITCSASSSISSN YLHWYQQKPDQSPKFLIYRTSNLASGVPSRFSGSGSGTDFTLTINSLEAEDAA TYYCQQASSIPRMFTFGQGTKLEIKGSTSGSGKPGSGEGSTKGEVQLVQSGAE VKKPGASVKVSCKASGYTFTNYWMHWVRQRPGQGLEWMGEIDPSDNYAN YNQEFQGRVTITVDKSASTAYMELSSLRSEDTAVYYCARHDGYFGALDYWG QGTTVTVSSGSFVPVFLPAKRTTTPAPRPPTPAPTIASQPLSLRPEACRPAAGG AVHTRGLDFACDIYIWAPLAGTCGVLLLSLVITLYCRSKRSRLLHSDYMNMT PRRPGPTRKHYQPYAPPRDFAAYRSRVKFSRSADAPAYQQGQNQLYNELNLG RREEYDVLDKRRGRDPEMGGKPRRKNPQEGLYNELQKDKMAEAYSEIGMK GERRRGKGHDGLYQGLSTATKDTYDALHMQALPPRGSGATNFSLLKQAGDV EENPGPMRISKPHLRSISIQCYLCLLLNSHFLTEAGIHVFILGCFSAGLPKTEAN WVNVISDLKKIEDLIQSMHIDATLYTESDVHPSCKVTAMKCFLLELQVISLESG DASIHDTVENLIILANNSLSSNGNVTESGCKECEELEEKNIKEFLQSFVHIVQM FINTS |
| hFMC63-CAR | 25 | MALPVTALLLPLALLLHAARPLEDIQMTQSPSSLSASVGDRVTITCRASQDISK YLNWYQQKPGGAVKLLIYHTSRLHSGVPSRFSGSGSGTDYTLTISSLQPEDFA TYYCQQGNTLPYTFGGGTKVEIKGSTSGSGKPGSGEGSTKGEVTLKESGPVLV KPTETLTLTCTVSGFSLSDYGVSWIRQPPGKALEWLAVIWGSETTYYNSALKS RLTISKDTSKSQVVLTMTNMDPVDTATYYCAKHYYYGGSYAMDYWGQGTT VTVSSGSFVPVFLPAKRTTTPAPRPPTPAPTIASQPLSLRPEACRPAAGGAVHT RGLDFACDIYIWAPLAGTCGVLLLSLVITLYCRSKRSRLLHSDYMNMTPRRPG PTRKHYQPYAPPRDFAAYRSRVKFSRSADAPAYQQGQNQLYNELNLGRREE YDVLDKRRGRDPEMGGKPRRKNPQEGLYNELQKDKMAEAYSEIGMKGERR RGKGHDGLYQGLSTATKDTYDALHMQALPPRGSGATNFSLLKQAGDVEENP GPMRISKPHLRSISIQCYLCLLLNSHFLTEAGIHVFILGCFSAGLPKTEANWVN VISDLKKIEDLIQSMHIDATLYTESDVHPSCKVTAMKCFLLELQVISLESGDASI HDTVENLIILANNSLSSNGNVTESGCKECEELEEKNIKEFLQSFVHIVQMFINTS |
| FMC63-CAR | 26 | MALPVTALLLPLALLLHAARPLEDIQMTQTTSSLSASLGDRVTISCRASQDISK YLNWYQQKPDGTVKLLIYHTSRLHSGVPSRFSGSGSGTDYSLTISNLEQEDIA TYFCQQGNTLPYTFGGGTKLEITGSTSGSGKPGSGEGSTKGEVKLQESGPGLV APSQSLSVTCTVSGVSLPDYGVSWIRQPPRKGLEWLGVIWGSETTYYNSALKS RLTIIKDNSKSQVFLKMNSLQTDDTAIYYCAKHYYYGGSYAMDYWGQGTSV TVSSGSFVPVFLPAKRTTTPAPRPPTPAPTIASQPLSLRPEACRPAAGGAVHTR GLDFACDIYIWAPLAGTCGVLLLSLVITLYCRSKRSRLLHSDYMNMTPRRPGP TRKHYQPYAPPRDFAAYRSRVKFSRSADAPAYQQGQNQLYNELNLGRREEY DVLDKRRGRDPEMGGKPRRKNPQEGLYNELQKDKMAEAYSEIGMKGERRR GKGHDGLYQGLSTATKDTYDALHMQALPPRGSGATNFSLLKQAGDVEENPG PMRISKPHLRSISIQCYLCLLLNSHFLTEAGIHVFILGCFSAGLPKTEANWVNVI SDLKKIEDLIQSMHIDATLYTESDVHPSCKVTAMKCFLLELQVISLESGDASIH DTVENLIILANNSLSSNGNVTESGCKECEELEEKNIKEFLQSFVHIVQMFINTS |

**Table 9. Grouping of NOG mice**

| Group | | Number of mice | NK cell | Dose | Volume | Vehicle |
|---|---|---|---|---|---|---|
| G1 | Efficacy | 5 | Negative control | / | 200µl | PBS |
| G2 | Metabolism/toxicolog y | 5 | Negative control | 4E6 | 200µl | PBS |
| G3 | Efficacy | 5 | Blank NK | 4E6 | 200µl | PBS |
| G4 | Efficacy | 5 | FMC63-CAR | 4E6 | 200µl | PBS |
| G5 | Metabolism/toxicolog y | 20 | FMC63-CAR | 4E6 | 200µl | PBS |
| G6 | Efficacy | 5 | hFMC63-CAR | 4E6 | 200µl | PBS |
| G7 | Metabolism/toxicolog y | 20 | hFMC63-CAR | 4E6 | 200µl | PBS |
| G8 | Efficacy | 5 | Hab01-4-CAR | 4E6 | 200µl | PBS |
| G9 | Metabolism/toxicolog y | 20 | Hab01-4-CAR | 4E6 | 200µl | PBS |
| G10 | Efficacy | 5 | Hab01-11-CAR | 4E6 | 200µl | PBS |
| G11 | Metabolism/toxicolog y | 20 | Hab01-11-CAR | 4E6 | 200µl | PBS |

### Example 3. Preparation of Composition of CD19 CAR-NK Cells

The Hab01-4-CAR cells prepared in Example 2 were expanded. When the total cell amount in each closed device exceeded 1.3E10, the cells were harvested, washed, and cryopreserved on the day. Meanwhile, samples were taken and sent for detection of cell count, phenotype, mycoplasma, and cell function. If the number of CD3⁺ cells in the CD19 CAR-NK cell harvest exceeded 1%, a CD3+ cell removal step was required before harvest. The harvested CD19 CAR-NK cell product was resuspended in a cell storage solution (the components of the cell storage solution include: a compound electrolyte injection, a human serum albumin injection, and a cryopreservation solution), aliquoted, and cryopreserved at a cell cryopreservation density of no less than 1E6 viable cells/mL for subsequent use.

### Example 4. Clinical Preliminary Exploration Study of CD19 CAR-NK Cell Composition in Tumor

Two subjects were selected and enrolled, who separately had B-cell NHL and B-ALL. The regimen was divided into a screening period, bridging therapy (as needed), a pre-lymphodepletion period, lymphodepletion, assessment before infusion, multiple infusions, and a follow-up period. The investigator determined whether to administer multiple doses as planned according to the condition of the subjects.

The characteristics, distribution and follow-up time of the study subjects are summarized in Tables 10 and 11. All patients received reinfusion treatment with the CD19 CAR-NK (hereinafter referred to as CD19 CAR-NK) cells prepared in Example 3. A single-dose study was initially performed, and the enrolled subjects included subject 1 in the 1E7 cell/kg CD19 CAR-NK dose group and subject 2 in the 2E7 cell/kg CD19 CAR-NK dose group.

As of the data cutoff date, subjects 1 and 2 completed the dose-limiting toxicity (DLT) observation period (28 days after the first reinfusion of CD19 CAR-NK), during which no protocol-specified related toxicity (DLT) events occurred.

NHL subject 1 completed the first efficacy evaluation (28 days after the first reinfusion of CD19 CAR-NK), and in the main follow-up stage, received 1E7 cells/kg CD19 CAR-NK treatment after the third-line treatment and achieved remission (CR (complete response)/CMR (complete molecular biology response)). ALL subject 2 completed the first bone marrow efficacy evaluation (14 days after the first reinfusion of CD19 CAR-NK), and in the main follow-up stage, received 2E7 cells/kg CD19 CAR-NK after the second-line treatment. The primary bone marrow biopsy result on D14 showed bone marrow response (CRi), but the extramedullary lesions were still not evaluated, and on D28, the extramedullary lesion imaging results showed complete response (CR).

It can be seen that the CD19 CAR-NK composition has certain clinical therapeutic effects on B-ALL and NHL.

**Table 10. Summary of demographics and subject characteristics in the clinical preliminary exploration study of tumors (all subjects receiving CD19 CAR-NK reinfusion)**

| Subject No. | Age (years) | Gender | Ethnicity | Tumor type | Date of first diagnosis | Bone marrow involvement/ Extramedullary lesion | CLL staging (Rai staging) | NHL disease staging |
|---|---|---|---|---|---|---|---|---|
| 1 | 53 | Female | Han | NHL | 2020-12 | None | NA | Stage IV |
| 2 | 36 | Female | Han | ALL | 2017-08 | With extramedullary lesion | NA | NA |

| | | | | | | | | |
|---|---|---|---|---|---|---|---|---|
| Note: NR, not reported; NA, not applicable | | | | | | | | |

**Table 11. Individual efficacy evaluation list in the clinical preliminary exploration study of tumors (all subjects receiving CD19 CAR-NK reinfusion)**

| Subject No. | Tumor type | Visit | Date of overall evaluation | Overall efficacy evaluation results |
|---|---|---|---|---|
| 1 | NHL | Main follow-up period D28 | 2022-07-05 | CMR |
| | | Main follow-up period D60 | 2022-08-03 | CMR |
| | | Main follow-up period D90 | 2022-09-09 | CT in another hospital: CR |
| | | Main follow-up period D120 | 2022-10-20 | CMR |
| | | Main follow-up period D180 | 2022-11-28 | CMR |
| | | Main follow-up period D270 | 2023-03-09 | CMR |
| 2 | ALL | Main follow-up period D14 | 2022-12-02 | CR |
| | | Main follow-up period D28 | 2022-12-21 | CR |

### Example 5. B-Cell Killing of Composition of CD19 CAR-NK Cells

Systemic lupus erythematosus (SLE) is an autoimmune disease involving multiple organs, the main pathogenesis of which is autoimmune reaction caused by immune imbalance, and the most critical factor is the dysregulation of the activation of autoreactive B cells. CD19 serves as a characteristic antigen on the surface of B cells. Anti-CD19 chimeric antigen receptor NK cells (CD19 CAR-NK) can specifically kill B cells, which can provide a new approach for treating SLE.

To study the killing effect of CD19 CAR-NK on B cells, a co-incubation system of CD19 CAR-NK and control Mock-NK cells with B cells isolated from peripheral blood of patients with lupus erythematosus (SLE) was established in vitro, and the specific killing of B cells from patients with lupus erythematosus by NK cells was detected. Specifically, the CD19 CAR-NK and control Mock-NK cells were co-incubated with B cells isolated from peripheral blood of patients with lupus erythematosus to perform short-acting killing for 4 h and multiple-run long-acting killing. For the short-acting killing, co-incubation was performed for 4 h at effector-to-target ratios of 1:1 and 2:1; for each round of the multiple-run killing, co-incubation was performed for 24 h at an effector-to-target ratio of 1:1, and a total of 2 rounds of co-incubation experiments were performed. At the end point of each round of co-incubation, the ratio of NK cells to B cells in the co-cultured cells was detected by flow cytometry, and the remaining cell quantity was detected by AO/PI cell counting method. The killing efficiency of CD19 CAR-NK on B cells isolated from patients with lupus erythematosus was calculated by combining the flow cytometry results with the cell counting results. The killing efficiency was calculated using the following formula: $Killing efficiency % = \frac{BTheoretical number of cells - BRemaining number of cells}{BTheoretical number of cells} \times 100 %$ *Note: The theoretical number of B cells refers to the number of seeded cells at the beginning of co-incubation.*

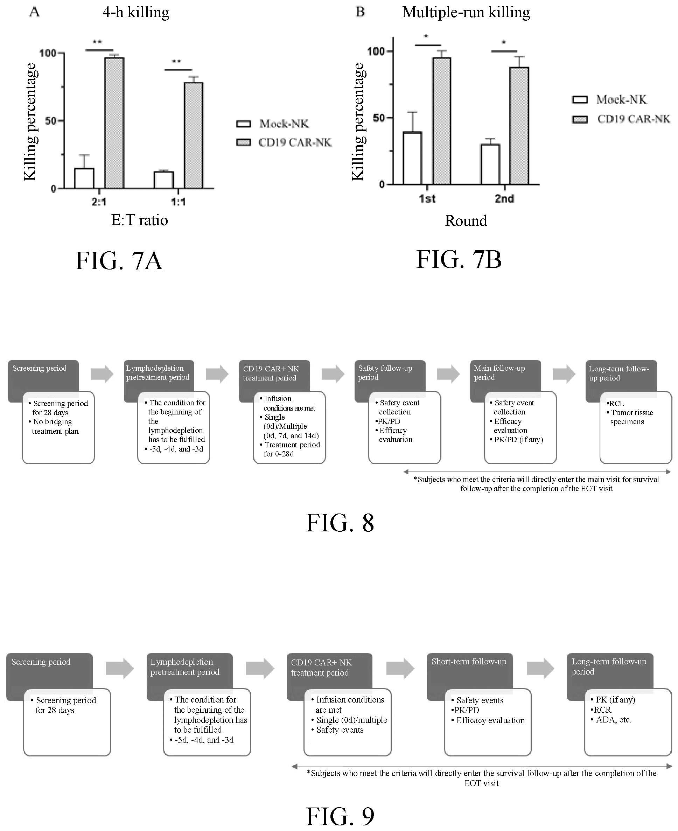

The results of the in vitro 4 h short-acting killing assay showed that, at an NK total cell-based effector-to-target ratio of 2:1, the killing efficiency of CD19 CAR-NK against SLE-B cells was significantly better than that of the control NK cells (Mock-NK) (96.89% vs 15.72%); at a low effector-to-target ratio of 1:1, CD19 CAR-NK still had a significant killing effect (78.52%), which was significantly better than that of the control Mock-NK (12.92%) (see FIG. 7A).

The results of the in vitro multiple-run killing assay showed that at the end of the first round of co-incubation, the mean killing efficiency of CD19 CAR-NK against SLE-B cells was 95.69%, which was significantly better than that of the control Mock-NK cells (39.43%); in the second round, the mean killing efficiency of CD19 CAR-NK against SLE-B cells was 88.47%, which was still significantly better than that of the Mock-NK cells (30.63%) (see FIG. 7B).

The above results show that the CD19 CAR-NK can effectively kill B cells from patients with lupus erythematosus, and the killing activity is significantly improved compared to that of the control Mock-NK cells.

The killing effects of CD19 CAR-NK on B cells from patients with myositis, scleroderma, and vasculitis were studied by a similar method. The results show that CD19 CAR-NK could effectively kill B cells from patients with myositis, scleroderma, and vasculitis, and the killing activity was significantly improved compared to that of the control Mock-NK cells.

### Example 6. Preliminary Clinical Protocol for Composition of CD19 CAR-NK Cells for Tumor Treatment

This mainly evaluated the safety and tolerability of CD19 CAR-NK in the treatment of subjects with recurrent/refractory non-Hodgkin's lymphoma, determined the maximum tolerated dose (MTD) and/or recommended phase II dose (RD), and meanwhile preliminarily evaluated the effectiveness of CD19 CAR-NK in the treatment of subjects with recurrent/refractory non-Hodgkin's lymphoma.

The enrollment of subjects with large B-cell lymphoma and follicular lymphoma was planned. The subjects should have received at least second-line treatment and had at least 1 measurable lesion according to Lugano 2014 Response Evaluation Criteria in Lymphoma (Cheson 2014).

The study was divided into two stages, i.e., dose escalation and dose expansion. In the dose escalation stage, a single dose escalation study was first performed, and then a multiple dose escalation study could be performed.

### Single dose escalation

Three dose groups were preset for the single dose escalation, with dose levels of (1) 1E9 CD19 CAR+ NK cells, (2) 2E9 CD19 CAR+ NK cells, and (3) 3E9 CD19 CAR+ NK cells in ascending order. A dose fluctuation of ±10% was permitted for each dose group. Subjects were scheduled to receive 1 infusion of CD19 CAR-NK only. Before the start of infusion of CD19 CAR-NK, the subjects in each dose group were evaluated by the investigator for suitability in receiving CD19 CAR-NK administration. After the DLT observation period (28 days after CD19 CAR-NK infusion) for the first enrolled subject in each dose group ended, data cleaning and safety data review were timely performed for this subject in this group, and the Safety Review Committee (SRC) discussed and decided whether the remaining subjects in this group could continue to be enrolled.

### Multiple dose escalation

The selection of dose groups for the multiple dose escalation and the administration frequency and dose of each dose group were determined by the sponsor based on the suggestions of the SRC after the completion of the single dose escalation. Two dose levels were planned, from low to high: (1) a dose that was lower than the RD dose confirmed in the single dose escalation study, (2) the RD dose confirmed in the single dose escalation study (a dose fluctuation of ±10% was permitted for each dose group). Subjects received up to 3 CD19 CAR-NK treatments once a week. Before each infusion of CD19 CAR-NK, the subjects in each dose group were evaluated by the investigator for suitability in receiving CD19 CAR-NK administration. After the DLT observation period (within 28 days after the first CD19 CAR-NK infusion) for the first enrolled subject in each dose group ended, data cleaning and safety data review were timely performed for this subject in this group, and the Safety Review Committee (SRC) discussed and decided whether to enroll the remaining 2 subjects in this group. The sponsor may also consider increasing dose groups to explore multiple administration at higher doses. DLT events and non-evaluable subjects for DLT were predefined. The DLT observation period was 28 days after the first administration of CD19 CAR-NK (the day of the first administration of CD19 CAR-NK was defined as 0d). For subjects evaluable for DLT, according to the "3 + 3" principle, the SRC determined the increase or decrease of the dose with reference to safety, PK, and PD data (if applicable), and the SRC could also make suggestions for exploring other dose levels, exploring other administration frequencies, etc., based on existing data at that time.

### Dose expansion stage

After completion of the study in the dose escalation stage, and determination of the MTD and/or RD of single/multiple administration by the SRC, the decision of whether to conduct the dose expansion study using a single-dose or multiple-dose regimen would be made by the sponsor. In the dose expansion stage, at least about 10 subjects (with up to 3 subjects previously treated with CD19-targeting CAR-T cell therapy) would be continuously enrolled at the determined RD dose level to further evaluate the safety and effectiveness of CD19 CAR-NK. Whether to continue to enroll to the maximum sample size would be determined based on the efficacy, safety, PK, immunogenicity, and other data of the about 10 subjects.

The study procedures were divided into: a screening period (-34 to -6d), a lymphodepletion pretreatment period (-5 to -3d), a CD19 CAR-NK treatment period (0 to +28d), a safety follow-up period (+28d to 3 months after the first CD19 CAR-NK administration day), a main follow-up period (after the end of the safety follow-up period to 2 years after the first CD19 CAR-NK administration day), and a long-term follow-up period (the completion of the main follow-up to 15 years after the first CD19 CAR-NK administration). Subjects were considered to be enrolled in the study once they had started receiving lymphodepletion pretreatment chemotherapy. The day of the first administration of CD19 CAR-NK was defined as 0d. From the start of the CD19 CAR-NK treatment period to any period thereafter, once disease progression occurred or the disease did not progress but a new anti-lymphoma therapy had been initiated, the subject should have an EOT visit, complete the examinations related to the EOT visit, and then directly enter survival follow-up. Subjects who withdrew from the study treatment due to other reasons would enter the subsequent follow-up period after completion of the EOT visit. Patients would withdraw from the trial if they actively requested to quit the long-term follow-up. Subjects other than those who had disease progression or had not progressed but were receiving new anti-lymphoma treatment were followed up for 15 years after the end of the 2-year main follow-up, i.e., once every year (± 2 months) long-term follow-up under the condition that long-term follow-up was met. In the study, PK tests would be performed at the central laboratory; laboratory tests would be performed at the study center; corresponding tests for efficacy evaluation were performed at the study center, and the efficacy evaluation was performed by the investigator (see FIG. 8).

The duration of the main study part of the study was 2 years after the last enrolled subject received the first dose of CD19 CAR-NK treatment, or the last subject in the group completed the clinical study, whichever occurred first. Overall end of the study was defined as: the end of the 15-year long-term follow-up for the last subject in the group.

### Example 7. Clinical Preliminary Exploration Study of CD19 CAR-NK Cell Composition in Autoimmune Disease

Three subjects were selected and enrolled, including 1 case of moderate to severe active systemic lupus erythematosus (SLE), 1 case of refractory granulomatosis with polyangiitis, and 1 case of systemic sclerosis. The regimen was divided into a screening period, a pre-lymphodepletion period, a lymphodepletion period, assessment before infusion, a CD19 CAR-NK treatment period, and a follow-up period.

The characteristics, distribution and follow-up time of the study subjects are summarized in Tables 12 and 13. All patients received CD19 CAR-NK cell reinfusion treatment. Each subject received 2 infusions of CD19 CAR-NK within 28 days, with the interval between the 2 infusions being 7 days. The dose groups for each subject are shown in Table 13.

As of the data cutoff date, all 3 subjects enrolled in this study completed the dose-limiting toxicity (DLT) observation period (28 days after the first reinfusion of CD19 CAR-NK), during which no protocol-specified related DLT events occurred.

Subjects with moderate to severe active SLE completed the first efficacy evaluation (28 days after the first reinfusion of CD19 CAR-NK), and achieved SRI-4 (SRI: SLE responder index) in the efficacy evaluations at W4, W8, W12, W20, and W24 in the main follow-up stage. In the W4, W8, and W12 efficacy evaluations of subjects with refractory granulomatosis with polyangiitis, the Birmingham vasculitis activity score (BVAS) decreased from 12 points at baseline to 5 points. For the subjects with systemic sclerosis, in the W4 and W8 efficacy evaluations, the mRSS (modified Rodnan skin score) skin score decreased from 13 points at baseline to 9 points at maximum, and the subjects were still in safety follow-up.

It can be seen that the CD19 CAR-NK composition has certain clinical therapeutic effects on autoimmune diseases, such as systemic lupus erythematosus, refractory granulomatosis with polyangiitis, and systemic sclerosis.

**Table 12. Summary of demographics and subject characteristics in the exploration study of autoimmune diseases (all subjects receiving CD19 CAR-NK reinfusion)**

| Subject No. | Age (years) | Gender | Ethnicity | Disease type | Date of first diagnosis | Complication |
|---|---|---|---|---|---|---|
| 1 | 31 | Female | Han | Moderate to severe active systemic lupus erythematosus | 2007-UK | Lupus encephalitis and lupus nephritis |
| 2 | 60 | Male | Han | Refractory granulomatosis with polyangiitis | 2001-03 | - |
| 3 | 55 | Female | Han | Systemic sclerosis | 2021-UK | Interstitial pneumonia, Sjogren's syndrome, and primary biliary cirrhosis |

| | | | | | | |
|---|---|---|---|---|---|---|
| Note: UK, unknown | | | | | | |

**Table 13. Individual efficacy evaluation list in the exploration study of autoimmune diseases (all subjects receiving CD19 CAR-NK reinfusion)**

| Dose group | Subject No. | Disease type | Visit | Date of overall evaluation | Overall efficacy evaluation results |
|---|---|---|---|---|---|
| 0.5× 10⁹ CAR⁺ NK cells, d0, d7 | | Moderate to severe active systemic lupus erythematosus | Main follow-up period W4 | 2024-02-19 | SRI-4 reached |
| | 1 | | Main follow-up period W8 | 2024-03-19 | SRI-4 reached |
| | | | Main follow-up period W12 | 2024-04-19 | SRI-4 reached |
| | | | Main follow-up | 2024-05-15 | SRI-4 reached |
| | | | period W16 | | |
| | | | Main follow-up period W20 | 2024-06-18 | SRI-4 reached |
| | | | Main follow-up period W24 | 2024-07-29 | SRI-4 reached |
| 1.0× 10⁹ CAR⁺ NK cells, d0, d7 | 2 | Refractory granulomatosis with polyangiitis | Main follow-up period W4 | 2024-05-26 | BVAS score 6 |
| | | | Main follow-up period W8 | 2024-06-29 | BVAS score 5 |
| | | | Main follow-up period W12 | 2024-07-26 | BVAS score 5 |
| 3.0× 10⁹ CAR⁺ NK cells, d0, d7 | 3 | Systemic sclerosis | Main follow-up period W4 | 2024-08-01 | mRSS score 9 |
| | | | Main follow-up period W8 | 2024-08-29 | mRSS score 9 |

### Example 8. Preliminary Clinical Protocol for Composition of CD19 CAR-NK Cells for Autoimmune Disease Treatment

This mainly evaluated the safety and tolerability of CD19 CAR-NK in the treatment of systemic lupus erythematosus (SLE), determined the maximum tolerated dose (MTD) and/or subsequent recommended dose (recommended phase II dose, RD), and meanwhile preliminarily evaluated the effectiveness, pharmacokinetic (PK) characteristics, immunogenicity, etc., of CD19 CAR-NK in treating SLE subjects.

The enrollment of subjects with moderate to severe refractory systemic lupus erythematosus was planned. The subjects were diagnosed with SLE at least 24 weeks before screening, and the 2019 European Alliance of Associations for Rheumatology (EULAR)/American College of Rheumatology (ACR) classification criteria for SLE were met at screening.

The study was divided into two stages, i.e., dose escalation and dose expansion. In the dose escalation stage, a single dose escalation study was first performed, and based on the results of the single dose escalation study, the MTD and/or subsequent recommended dose (RD) were determined, and whether the study needed to continue with a multiple dose escalation study was also determined; if the single dose was sufficient to meet the therapeutic requirements, the multiple dose escalation study could be skipped, and the recommendations were directly given to the dose expansion stage according to the MTD and/or RD dose of the single dose.

### Single dose escalation

Four dose groups were preset for the single dose escalation, with dose levels of (1) 0.5E9 CD19 CAR+ NK cells, (2) 3E9 CD19 CAR+ NK cells, (3) 6E9 CD19 CAR+ NK cells, and (4) 9E9 CD19 CAR+ NK cells in ascending order. A dose fluctuation of ±10% was permitted for each dose group. In the single dose escalation stage, accelerated titration combined with the "3 + 3" design was used to guide dose escalation. In the single dose escalation stage, the subjects were scheduled to receive 1 infusion of CD19 CAR-NK only. Before the start of infusion of CD19 CAR-NK, the subjects in each dose group were evaluated by the investigator for suitability in receiving CD19 CAR-NK administration.

### Multiple dose escalation

In the multiple dose escalation stage, the "3 + 3" design was used to guide dose escalation. The selection of dose groups for the multiple dose escalation and the administration frequency of each dose group were determined by the sponsor based on the suggestions of the SRC after the completion of the single dose escalation. Two dose levels were planned, from low to high: (1) a dose that was lower than the RD dose confirmed in the single dose escalation study, (2) the RD dose confirmed in the single dose escalation study (a dose fluctuation of ±10% was permitted for each dose group). Subjects received up to 2 CD19 CAR-NK treatments once a week. Before each infusion of CD19 CAR-NK, the subjects in each dose group were evaluated by the investigator for suitability in receiving CD19 CAR-NK administration.

DLT events and non-evaluable subjects for DLT were predefined. The DLT observation period was 28 days after the last administration of CD19 CAR-NK. For subjects evaluable for DLT, the SRC would determine the increase or decrease of the dose with reference to safety, PK, and PD data (if applicable), and the SRC may also make suggestions for exploring other dose levels, exploring other administration frequencies, etc., based on existing data at that time.

### Dose expansion stage

After completion of the study in the dose escalation stage, and determination of the MTD and/or RD of single/multiple administration by the SRC, the decision of whether to conduct the dose expansion study using a single-dose or multiple-dose regimen would be made by the sponsor. In the dose expansion stage, about 10 subjects would be continuously enrolled at the determined RD dose level to further evaluate the safety and effectiveness of CD19 CAR-NK. Subsequently, whether to continue to enroll to the maximum sample size would be determined based on the efficacy, safety, PK, immunogenicity, and other data of the about 10 subjects.

The study procedures were divided into: a screening period (-34 to -6d), a lymphodepletion pretreatment period (-5 to -3d), a CD19 CAR-NK treatment period (0 to +28d after the last administration day of F01), a short-term follow-up (W8 to W96), and a long-term follow-up period (W96 to 15 years). Subjects were considered to be enrolled in the study once they had started receiving lymphodepletion pretreatment chemotherapy. The day of the first administration of CD19 CAR-NK was defined as 0d. Discontinuation of study treatment due to any reason after the first CD19 CAR-NK infusion required completion of the EOT visit. If subjects did not withdraw from the study, they would enter the subsequent follow-up period after completing the EOT visit. If the subjects experienced disease deterioration/recurrence or started the protocol-prohibited rescue therapy in advance, a survival follow-up would be performed directly after the completion of the EOT visit, and the information about the rescue therapy for the subjects would be collected. Patients would withdraw from the trial if they actively requested to quit the short-term follow-up. Long-term follow-up would be performed for up to 15 years after the end of the 2-year short-term follow-up, except for subjects who experienced disease deterioration/recurrence and needed to receive rescue therapy prohibited by the protocol. If a subject developed a second primary malignancy (SPM), they should be followed up for life. In the study, PK/PD tests would be performed at the central laboratory; laboratory tests would be performed at the study center; corresponding tests for efficacy evaluation were performed at the study center, and the disease evaluation was performed by the investigator (see FIG. 9).

In the study, any adverse events and serious adverse events (including AESIs) that occurred from the start of the lymphodepletion pretreatment chemotherapy to 12 weeks after the first infusion of the CD19 CAR-NK product or from the start of the protocol-prohibited rescue therapy (whichever occurs first) would be collected and recorded. Only adverse events and serious adverse events (including AESIs) related to investigator evaluation and study treatment were collected 12 weeks after the first infusion of the CD19 CAR-NK product. The duration of the main study part of the study was 2 years after the last enrolled subject received the first dose of CD19 CAR-NK treatment, or the last subject in the group completed the clinical study, whichever occurred first. Overall end of the study was defined as: the end of the 15-year long-term follow-up for the last subject in the group.

The teachings of all patents, published applications, and references cited herein are incorporated by reference in their entirety.

While exemplary examples have been specifically shown and described, it will be understood by those skilled in the art that various changes in forms and details may be made therein without departing from the scope of the embodiments encompassed by the appended claims.

## Claims

1. A pharmaceutical composition, comprising an immune effector cell and a pharmaceutically acceptable carrier, wherein the immune effector cell comprises a chimeric antigen receptor targeting CD19, the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3, wherein:
(1) the HCDR1-3 have the sequences set forth in SEQ ID NOs: 5-7, respectively, and the LCDR1-3 have the sequences set forth in SEQ ID NOs: 8-10, respectively; or
(2) the HCDR1-3 have 0-3 mutations compared with each corresponding CDR in group (1), respectively, and/or the LCDR1-3 have 0-3 mutations compared with each corresponding CDR in group (1), respectively.

2. The pharmaceutical composition according to claim 1, wherein
(1) the VH has the sequence set forth in SEQ ID NO: 20, and the VL has the sequence set forth in SEQ ID NO: 17; or
(2) the VH has a sequence having at least 80% identity or at most 25 mutations compared with the corresponding VH in group (1), and the VL has a sequence having at least 80% identity or at most 20 mutations compared with the corresponding VL in group (1).

3. The pharmaceutical composition according to claim 1 or 2, wherein the chimeric antigen receptor has the sequence set forth in SEQ ID NO: 23, or has a sequence having at least 80% identity or at most 120 amino acid mutations compared with SEQ ID NO: 23.

4. The pharmaceutical composition according to any one of claims 1-3, wherein the pharmaceutical composition is cryopreserved immune cells.

5. The pharmaceutical composition according to claim 4, wherein the pharmaceutically acceptable carrier is a cell storage solution.

6. The pharmaceutical composition according to claim 4 or 5, wherein the immune effector cell cryopreservation density in the cryopreserved immune cells is no less than 1 × 10⁵ viable cells/mL; preferably, the immune effector cell cryopreservation density in the cryopreserved immune cells is no less than 1 × 10⁶ viable cells/mL.

7. The pharmaceutical composition according to any one of claims 1-5, wherein the pharmaceutical composition is an injection.

8. The pharmaceutical composition according to any one of claims 1-6, wherein the proportion of CD3⁺ cells in the pharmaceutical composition is no more than 5%; preferably, the proportion of CD3⁺ cells in the immune effector cells is no more than 3%; more preferably, the proportion of CD3⁺ cells in the immune effector cells is no more than 1%.

9. A method for treating a disease, comprising administering to a subject in need the pharmaceutical composition according to any one of claims 1-8, wherein the disease is a B cell-driven disease.

10. The method according to claim 9, wherein the disease is a CD19-positive B cell-driven disease.

11. The method according to claim 9 or 10, wherein the disease is a tumor.

12. The method according to claim 11, wherein the tumor is a CD19-related tumor; preferably, the CD19-related tumor is a B cell malignancy.

13. The method according to claim 12, wherein the CD19-related tumor is a CD19-positive B cell malignancy; preferably, the CD19-positive B cell malignancy is a recurrent or refractory CD19-positive B cell malignancy.

14. The method according to claim 12 or 13, wherein the CD19-related tumor is selected from one or more of acute lymphocytic leukemia (ALL), chronic lymphocytic leukemia (CLL), non-Hodgkin's lymphoma (NHL), diffuse large B cell lymphoma (DLBCL), and marginal zone B-cell non-Hodgkin's lymphoma (MZL).

15. The method according to any one of claims 11-14, wherein the subject has previously received at least second-line treatment.

16. The method according to any one of claims 11-15, wherein the subject has at least 1 measurable lesion according to Lugano 2014 Response Evaluation Criteria in Lymphoma (Cheson 2014).

17. The method according to claim 9 or 10, wherein the disease is an autoimmune disease.

18. The method according to claim 17, wherein the autoimmune disease is selected from one or more of systemic lupus erythematosus (SLE), inflammatory bowel disease (IBD), multiple sclerosis (MS), rheumatoid arthritis (RA), type I diabetes (T1DM), Sjogren's syndrome, myositis, vasculitis, and scleroderma.

19. A method for treating an autoimmune disease, comprising administering to a subject in need an immune effector cell comprising a chimeric antigen receptor targeting CD19, wherein the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3, wherein:
(1) the HCDR1-3 have the sequences set forth in SEQ ID NOs: 5-7, respectively, and the LCDR1-3 have the sequences set forth in SEQ ID NOs: 8-10, respectively; or
(2) the HCDR1-3 have 0-3 mutations compared with each corresponding CDR in group (1), respectively, and/or the LCDR1-3 have 0-3 mutations compared with each corresponding CDR in group (1), respectively.

20. The method according to claim 19, wherein
(1) the VH has the sequence set forth in SEQ ID NO: 20, and the VL has the sequence set forth in SEQ ID NO: 17; or
(2) the VH has a sequence having at least 80% identity or at most 25 mutations compared with the corresponding VH in group (1), and the VL has a sequence having at least 80% identity or at most 20 mutations compared with the corresponding VL in group (1).

21. The method according to claim 19 or 20, wherein the chimeric antigen receptor has the sequence set forth in SEQ ID NO: 23, or has a sequence having at least 80% identity or at most 120 amino acid mutations compared with SEQ ID NO: 23.

22. The method according to any one of claims 19-21, wherein the autoimmune disease is selected from one or more of systemic lupus erythematosus (SLE), inflammatory bowel disease (IBD), multiple sclerosis (MS), rheumatoid arthritis (RA), type I diabetes (T1DM), Sjogren's syndrome, myositis, vasculitis, and scleroderma.

23. The method according to any one of claims 17-22, wherein the subject is diagnosed with SLE at least 24 weeks before screening.

24. The method according to any one of claims 17-23, wherein the subject meets the 2019 European Alliance of Associations for Rheumatology (EULAR)/American College of Rheumatology (ACR) classification criteria for SLE at screening.

25. The method according to any one of claims 11-24, wherein administration of the pharmaceutical composition is a single administration or multiple administration;
optionally, the administration is performed once a week; and
optionally, the single administration is 1 administration; the multiple administration is 2-6 administrations; preferably, the multiple administration is 2, 3, 4, 5, and 6 administrations.

26. The method according to any one of claims 11-25, wherein the pharmaceutical composition is administered at 1 × 10⁴ cells/Kg to 1 × 10⁹ cells/Kg each time;
preferably, the pharmaceutical composition is administered at 1 × 10⁶ cells/Kg to 9 × 10⁸ cells/Kg each time;
preferably, the pharmaceutical composition is administered at 1 × 10⁶ cells/Kg to 9 × 10⁶ cells/Kg, 1 × 10⁷ cells/Kg to 9 × 10⁷ cells/Kg, or 1 × 10⁸ cells/Kg to 9 × 10⁸ cells/Kg each time;
more preferably, the pharmaceutical composition is administered at 5 × 10⁶ cells/Kg, 6 × 10⁶ cells/Kg, 7 × 10⁶ cells/Kg, 8 × 10⁶ cells/Kg, 9 × 10⁶ cells/Kg, 1 × 10⁷ cells/Kg, 2 × 10⁷ cells/Kg, 3 × 10⁷ cells/Kg, 4 × 10⁷ cells/Kg, 5 × 10⁷ cells/Kg, 6 × 10⁷ cells/Kg, 7 × 10⁷ cells/Kg, 8 × 10⁷ cells/Kg, 9 × 10⁷ cells/Kg, 1 × 10⁸ cells/Kg, 2 × 10⁸ cells/Kg, 3 × 10⁸ cells/Kg, 4 × 10⁸ cells/Kg, 5 × 10⁸ cells/Kg, 6 × 10⁸ cells/Kg, 7 × 10⁸ cells/Kg, 8 × 10⁸ cells/Kg, or 9 × 10⁸ cells/Kg each time.

27. Use of a pharmaceutical composition or an immune effector cell in the preparation of a medicament for treating a disease, wherein the pharmaceutical composition is the pharmaceutical composition according to any one of claims 1-8, and the immune cell is the immune cell according to any one of claims 1-8 or claims 19-21;
the disease is a B cell-driven disease; and
the treatment comprises administering to a subject in need the pharmaceutical composition or the immune effector cell.

28. A pharmaceutical composition or an immune effector cell for treating a disease, wherein the pharmaceutical composition is the pharmaceutical composition according to any one of claims 1-8, and the immune cell is the immune cell according to any one of claims 1-8 or claims 19-21;
the disease is a B cell-driven disease; and
the treatment comprises administering to a subject in need the pharmaceutical composition or the immune effector cell.
